# EUROPÄISCHE PATENTSCHRIFT

(11) **EP 3 691 723 B1**
(45) Veröffentlichungstag und Bekanntmachung des Hinweises auf die Patenterteilung: **17.08.2022**
(21) Anmeldenummer: 18783376.9
(22) Anmeldetag: 24.09.2018
(51) Int. Cl.: A61M 16/00, A61M 16/20

(54) **BEATMUNGSVORRICHTUNG MIT AUTOMATISIERTER ERFASSUNG EINES FEHLERS EINES DURCHFLUSSSENSORS UNTER BERÜCKSICHTIGUNG VON SPONTANATMUNG**
VENTILATOR WITH AUTOMATIC DETECTION OF A FAULT IN A FLOW SENSOR, TAKING INTO ACCOUNT SPONTANEOUS BREATHING
DISPOSITIF RESPIRATOIRE COMPRENANT LA DÉTECTION AUTOMATIQUE D'UNE ERREUR D'UN CAPTEUR DE DÉBIT EN TENANT COMPTE D'UNE RESPIRATION SPONTANÉE

(30) Priorität: 06.10.2017 DE 102017217858
(43) Veröffentlichungstag der Anmeldung: 12.08.2020
(73) Patentinhaber: Hamilton Medical AG, 7402 Bonaduz (CH)
(72) Erfinder: SCHRANZ, Christoph, 7306 Fläsch (CH); WOLF, Dominik, 7000 Chur (CH); NOVOTNI, Dominik, 7000 Chur (CH)
(74) Vertreter: Ruttensperger Lachnit Trossin Gomoll
(86) Internationale Anmeldenummer: PCT/EP2018/075813
(87) Internationale Veröffentlichungsnummer: WO 2019/068496

(56) Entgegenhaltungen:
- WO-A1-03/055552
- WO-A1-2017/037152
- US-A1- 2008 257 350

## Beschreibung

Die vorliegende Erfindung betrifft eine Beatmungsvorrichtung zur künstlichen Beatmung eines Patienten mit
- einer Beatmungsgasquelle,
- einer zwischen der Beatmungsgasquelle und einem patientenseitigen, proximalen Ende verlaufenden Beatmungsleitungsanordnung,
- einer Ventilanordnung, umfassend ein Inspirationsventil und ein Exspirationsventil,
- einer Durchflusssensoranordnung zur quantitativen Erfassung eines Gasflusses in der Beatmungsleitungsanordnung, umfassend einen weiter vom patientenseitigen Ende der Beatmungsleitungsanordnung entfernt angeordneten distalen Durchflusssensor und einen näher beim patientenseitigen Ende der Beatmungsleitungsanordnung gelegenen proximalen Durchflusssensor,
- eine Drucksensoranordnung zur quantitativen Erfassung eines Gasdrucks von in der Beatmungsleitungsanordnung strömendem Gas,
- einer Druckveränderungsanordnung zur Veränderung des Gasdrucks des in der Beatmungsleitungsanordnung strömenden Gases, und mit
- einer Steuereinrichtung, welche wenigstens dazu ausgebildet ist,
   - den Betrieb der Druckveränderungsanordnung auf Grundlage von Messsignalen des proximalen Durchflusssensors zu steuern, und
   - in Abhängigkeit von Messsignalen des proximalen Durchflusssensors und des distalen Durchflusssensors auf einen Fehler des proximalen Durchflusssensors zu schließen.

Eine solche Beatmungsvorrichtung ist aus der WO 2017/037152 A1 bekannt.

Aus der WO 03/055552 A1 ist eine Beatmungsvorrichtung bekannt, deren Steuereinrichtung dazu ausgebildet ist, aus einem anwachsenden betragsmäßigen Unterschied zwischen den von distalem und proximalem Flusssensor erfassten Fluss- oder Volumenwerten auf eine Leckage in der Beatmungsleitung zu schließen.

Aus der US 2008/257350 A1 ist ein Kalibrationsverfahren für Sensoren in einer Beatmungsvorrichtung bekannt.

Als eine weitere Beatmungsvorrichtung ist beispielsweise im Markt das Produkt "SERVO-U" der Firma Maquet bekannt. Diese bekannte Beatmungsvorrichtung verwendet einen distalen Durchflusssensor im Inneren eines Beatmungsgeräts. An dem Beatmungsgerät ist das distale Ende einer Beatmungsschlauchanordnung der Beatmungsleitungsanordnung angeschlossen. Weiter verwendet die bekannte Beatmungsvorrichtung einen proximalen Durchflusssensor in Form eines Heißdrahtanemometers in einem Y-Verbindungsstück. Das Y-Verbindungsstück verbindet auf seiner der Beatmungsgasquelle zugewandten Seite ein Schlauchpaar aus einem inspiratorischen Beatmungsschlauch und einen vom inspiratorischen Beatmungsschlauch gesondert ausgebildeten exspiratorischen Beatmungsschlauch mit einer auf der dem Patienten zugewandten Seite des Beatmungsschlauchs angeordneten, zum Patienten hin führenden Beatmungsleitung. Die Bedienungsanleitung zu dieser bekannten Beatmungsvorrichtung gibt ohne nähere Spezifikation an, dass die Ausgaben von internen Druck- und Flusssensoren mit dem Messergebnis des proximalen Sensors in dem Y-Verbindungsstück verglichen werden und der proximale Sensor deaktiviert wird, wenn zwischen den zum Vergleich herangezogenen Werten eine signifikante Abweichung festgestellt wird.

Die Beatmungsvorrichtung "SERVO-U" ist jedoch nicht gattungsgemäß, da deren Steuereinrichtung nicht zur Steuerung des Betriebs der Druckveränderungsanordnung auf Grundlage von Messsignalen des proximalen Durchflusssensors ausgebildet ist.

Als gattungsgemäße Beatmungsvorrichtungen sind beispielsweise Beatmungsvorrichtungen der Anmelderin bekannt, die im Markt unter den Bezeichnungen "Hamilton-S1", "Hamilton-G1" und "Hamilton-C3" angeboten und vertrieben werden. Diese sind in der Lage gemäß dem aus dem Hause der Anmelderin bekannten Beatmungsmodus "Adaptive Pressure Ventilation (APV)" zu arbeiten, bei welchem die Steuereinrichtung in Abhängigkeit vom Messsignal des proximalen Durchflusssensors, also abhängig vom erfassten proximalen Fluss des Beatmungsgases, den Druck des Beatmungsgases in der Beatmungsleitungsanordnung so verändert, dass das dem Patienten verabreichte Gasvolumen einen vorbestimmten Zielwert oder Zielwertebereich erreicht beziehungsweise beibehält. Andere fachübliche Bezeichnungen für den APV-Modus von Beatmungsvorrichtungen der Anmelderin sind PC-CMVa und PC-IMV (jeweils nach Chatburn) oder PRVC ("pressure regulated volume control").

Die Steuereinrichtungen der gattungsgemäßen Beatmungsvorrichtungen vergleichen aus Messsignalen des distalen und des proximalen Durchflusssensors ermittelte Werte eines Beatmungsgasvolumens und schließen dann auf einen Fehler des proximalen Durchflusssensors, wenn sich das auf Grundlage von Messsignalen des proximalen Durchflusssensors ermittelte proximale Beatmungsgasvolumen und das von Messsignalen des distalen Durchflusssensors ermittelte distale Beatmungsgasvolumen in einer anderen als der für den jeweils herrschenden Betriebszustand zu erwartenden bzw. zulässigen Weise unterscheiden. Der abhängig vom Betriebszustand erwartete oder zulässige Unterschied der Beatmungsgasvolumina kann durch Schwellenwert-Kennwerte oder -Kennfelder berücksichtigt werden, in welchen in einem Datenspeicher der Steuereinrichtung wenigstens ein Unterschiedsschwellenwert zur Fehlererkennung hinterlegt ist, insbesondere als Funktion anderer Betriebsparameter hinterlegt ist.

Nachteilig an der bekannten gattungsgemäßen Beatmungsvorrichtung und der dort offenbarten, allein auf die Messsignale der Durchflusssensoren gestützten Fehlererfassung ist, dass während der Beatmung von Patienten trotz vorliegender Funktionstüchtigkeit der Messtechnik an der Beatmungsvorrichtung Situationen eintreten können, die die Messsignale des distalen und des proximalen Durchflusssensors so beeinflussen, dass ein Vergleich derselben oder ein Vergleich von daraus ermittelten Werten ohne weitere Maßnahmen zu einer falsch-positiven Fehlererfassung führt. Grundsätzlich ist vorauszuschicken, dass der vom distalen Durchflusssensor erfasste Beatmungsgasflusswert oder/und der daraus ermittelte Beatmungsgasvolumenwert stets betragsmäßig größer ist bzw. sind als der entsprechende vom proximalen Durchflusssensor erfasste Flusswert oder/und der daraus ermittelte Volumenwert. Dies liegt zu einem Teil an Elastizitäten in dem zwischen distalem und proximalem Durchflusssensor liegenden Abschnitt der Beatmungsleitungsanordnung. Ein Anteil des Gasstroms, welcher vom distalen Durchflusssensor erfasst wird, dient der Dehnung der elastischen Abschnitte der Beatmungsleitungsanordnung und erreicht den proximalen Durchflusssensor nicht. Dies liegt zu einem weiteren Teil an Undichtigkeiten des Exspirationsventils, wegen welcher ein Anteil am gesamten Beatmungsgasstrom als Querstrom in einem strömungsmechanischen Kurzschluss vom Inspirationsventil zum Exspirationsventil strömen kann, ohne den Patienten und ohne den proximalen Durchflusssensor zu erreichen. Auch der als Querstrom für die Beatmung eines Patienten verlorene Gasflussanteil wird vom distalen, nicht jedoch vom proximalen Durchflusssensor erfasst.

Es hat sich nun gezeigt, dass eine tatsächliche Beeinträchtigung der Funktionstüchtigkeit des proximalen Durchflusssensors durch sich darin ansammelnde Flüssigkeit - sei es als Kondensat aus der Beatmungsluft oder sei es alternativ oder zusätzlich durch Speichel oder Körpersekret des Patienten - zu einer signifikanten Verfälschung des Erfassungsergebnis des proximalen Durchflusssensors führt. Genauer wird unter dem Einfluss der sich im proximalen Durchflusssensor ansammelnden Flüssigkeit üblicherweise ein höherer Beatmungsgasfluss erfasst und daraus ein höheres Beatmungsgasvolumen ermittelt als tatsächlich vorhanden ist.

Der in der Regel von Flüssigkeit stets unbeeinflusste distale Durchflusssensor erfasst nach wie vor den korrekten Beatmungsgasfluss, sodass sich aufgrund der fehlerhaften Erfassung durch den proximalen Durchflusssensor die erfassten Flusswerte beider Sensoren und ebenso die aus den Flusswerten - in der Regel durch Integration über die Zeit - ermittelten Volumenwerte betragsmäßig annähern.

Dann nämlich, wenn, wie etwa in dem oben genannten Betriebsmodus "APV" das proximale Beatmungsgasvolumen durch Veränderung des Beatmungsgasdrucks gesteuert bzw. geregelt wird, führt eine Zunahme des erfassten proximalen Beatmungsgasflusses und folglich des daraus ermittelten Beatmungsgasvolumens steuerungs- bzw. regelungsbedingt zu einer Absenkung des Beatmungsgasdruckes, um den als erhöht erfassten proximalen Flusswert oder/und den daraus als erhöht ermittelten Volumenwert wieder auf sein ursprüngliches Soll-Niveau zu senken.

Die Steuereinrichtung hält somit das aus dem erfassten proximalen Beatmungsgasfluss ermittelte proximale Beatmungsgasvolumen durch Verminderung des Beatmungsgasdrucks konstant. Als Folge des verringerten Drucks des Beatmungsgases sinkt jedoch der vom distalen Durchflusssensor erfasste Fluss und das daraus ermittelte Volumen, sodass es wiederum zu einer betragsmäßigen Annäherung der vom distalen und vom proximalen Durchflusssensor erfassten Flusswerte oder/und der daraus ermittelten Volumenwerte kommt.

Die betragsmäßige Annäherung der erfassten Flusswerte oder/und der daraus ermittelten Volumenwerte von distalem und proximalem Durchflusssensor ist somit zwar in den meisten Fällen ein Indiz für einen in seiner Funktionalität beeinträchtigten proximalen Durchflusssensor, jedoch nicht in allen Fällen. Allerdings ist auch eine geringe Anzahl von falsch-positiven Fehlermeldungen oder Alarmen bei der künstlichen Beatmung von Patienten unerwünscht.

Es ist daher Aufgabe der vorliegenden Erfindung, Beatmungsvorrichtungen der gattungsgemäßen Art derart weiterzubilden, dass ein Fehler des proximalen Durchflusssensors mit noch größerer Sicherheit als bisher erkannt werden kann und die oben beschriebenen falsch-positiven Fehlererfassungen vermieden werden können.

Diese Aufgabe wird gelöst durch eine Beatmungsvorrichtung mit allen Merkmalen des Anspruchs 1. Bei dieser Beatmungsvorrichtung ist die Steuereinrichtung dazu ausgebildet, in Abhängigkeit von Messsignalen des proximalen Durchflusssensors und des distalen Durchflusssensors, und zwar aus einer betragsmäßigen Annäherung der erfassten Flusswerte oder/und der daraus ermittelten Volumenwerte von distalem und proximalem Durchflusssensor, auf einen Fehlerkandidaten des proximalen Durchflusssensors zu schließen und erst mit zeitlicher Verzögerung nach dem Erkennen des Fehlerkandidaten auf einen Fehler des proximalen Durchflusssensors zu schließen, wobei die Steuereinrichtung dazu ausgebildet ist, den Fehlerkandidaten zu verwerfen, wenn während einer Verifizierungsphase, welche mit oder nach dem Erkennen des Fehlerkandidaten beginnt, wenigstens ein ein Ausmaß einer Spontanatmungsaktivität des Patienten berücksichtigendes Verwerfen-Kriterium zum Verwerfen des Fehlerkandidaten erfüllt wird.

Dann, wenn ein Verwerfen-Kriterium während einer Verifizierungsphase erfüllt wird, kann der zuvor erkannte Fehlerkandidat verworfen werden, sodass der proximale Durchflusssensor nach wie vor als korrekt arbeitend beurteilt wird und die Steuereinrichtung keine weitere Maßnahme auslöst, sondern die Beatmungsvorrichtung weiter gemäß der gewählten Betriebsart betreibt.

Klinische Versuche haben gezeigt, dass eine Situation, die zur Erkennung eines Fehlerkandidaten führt, also die betragsmäßige Abnahme des Unterschieds zwischen den ausgehend von vom distalen Durchflusssensor und vom proximalen Durchflusssensor gemessenen Beatmungsgasflusswerten ermittelten Beatmungsgasvolumenwerten, auch durch Spontanatmung des Patienten hervorgerufen werden kann. Durch einen vom Patienten ausgelösten bzw. getriggerten aktiven Atemhub kann der vom proximalen Durchflusssensor gemessene Beatmungsgasflusswert oder/und der daraus ermittelte Beatmungsgasvolumenwert gegenüber den entsprechenden Werten von rein mandatorisch durch die Beatmungsvorrichtung getriggerten Atemhüben erhöht sein. Als Folge davon wird die Steuereinrichtung die Druckvorgabe für das inspiratorische Beatmungsgas in der Beatmungsleitungsanordnung senken, um den vom proximalen Durchflusssensor gemessenen Wert oder/und den daraus ermittelten Wert auf seinen Sollwert zurückzuführen. Durch den verringerten Beatmungsgasdruck sinkt der am internen Durchflusssensor gemessene distale inspiratorische Beatmungsgasfluss sowie das daraus ermittelte distale inspiratorische Beatmungsgasvolumen, sodass der betragsmäßige Unterschied zwischen den vom distalen Durchflusssensor und vom proximalen Durchflusssensor gemessenen Beatmungsgasflüssen oder/und den daraus jeweils ermittelten Beatmungsgasvolumina abnimmt.

Um eine durch Spontanatmung bzw. aktive Atemhübe ausgelöste falsch-positive Erkennung eines Fehlers des proximalen Durchflusssensors durch die Steuereinrichtung zu vermeiden, ist daher erfindungsgemäß vorgesehen, dass das wenigstens eine Verwerfen-Kriterium Spontanatmung des an die Beatmungsvorrichtung angeschlossenen Patienten berücksichtigt. Beispielsweise kann das wenigstens eine Verwerfen-Kriterium von einer Anzahl von durch den Patienten ausgelösten aktiven Atemhüben abhängig sein bzw. eine solche Anzahl berücksichtigen. Als "Anzahl" im Sinne der vorliegenden Erfindung gilt auch ein Anteil von aktiven Atemhüben an einer Gesamtanzahl von Atemhüben.

Das Verwerfen-Kriterium kann ein vorbestimmtes Verwerfen-Kriterium sein, d. h. es kann in der Steuereinrichtung oder in einem mit der Steuereinrichtung zusammenarbeitenden Datenspeicher vorab gespeichert sein und im Bedarfsfall aus dem Datenspeicher ausgelesen werden. Ebenso ist jedoch denkbar, dass das Verwerfen-Kriterium abhängig von Betriebsparametern der Beatmungsvorrichtung sowie von Patientendaten bzw. Patientenparametern während des Betriebs der Beatmungsvorrichtung ermittelt wird. Die Verwendung eines vorbestimmten Verwerfen-Kriteriums ist bevorzugt. Dabei soll auch ein solches Verwerfen-Kriterium im Sinne der vorliegenden Anmeldung als vorbestimmt gelten, das zwar durch die Steuereinrichtung abhängig von eingestellten Betriebsparametern der Beatmungsvorrichtung oder/und von eingestellten Patientendaten individuell für den gewählten Betrieb oder/und den zu beatmenden Patienten ermittelt wird, jedoch nach seiner Ermittlung in einem Datenspeicher abgespeichert wird und dort zur weiteren Verwendung während des Beatmungsbetriebs bereitsteht. Das zur Vorbestimmtheit des Verwerfen-Kriteriums Gesagte gilt auch für die Vorbestimmtheit aller anderen in dieser Anmeldung erwähnten Parameter.

Für den Beginn oder/und die Dauer der Verifizierungsphase gilt daher hinsichtlich Ermittlung bzw. Vorbestimmung das gleiche wie für das Verwerfen-Kriterium. Bevorzugt ist die Verifizierungsphase hinsichtlich Beginn und Dauer vorbestimmt, es soll jedoch nicht ausgeschlossen sein, dass ihr Beginn oder/und ihre Dauer individuell für einen erkannten Fehlerkandidaten abhängig von den zuvor genannten möglichen Parametern: Betriebsparameter der Beatmungsvorrichtung oder/und Zustandsparameter des Patienten (Patientendaten), ermittelt wird.

Die Verifizierungsphase kann eine als Zeitdauer angegebene Phase sein. Bevorzugt ist die Verifizierungsphase eine Anzahl an Atemhüben. Ein Atemhub ist dabei ein einen Inspirationsvorgang und einen Exspirationsvorgang umfassender Respirationsvorgang eines Patienten, welcher sich zeitlich zwischen den Auslösezeitpunkten (in der Fachwelt auch als "Triggerzeitpunkte" bezeichnet) zweier unmittelbar aufeinanderfolgender Inspirationsvorgänge erstreckt, und welcher zur Beatmung eines Patienten periodisch wiederholt wird.

Durch das Verwerfen des Fehlerkandidaten, sobald das wenigstens eine Verwerfen-Kriterium während der Verifizierungsphase erfüllt wird, kann eine falsch-positive Fehlermeldung oder ein falsch-positiver Alarm aufgrund einer Spontanatmung frühzeitig vermieden werden. Die Ausgabe einer Fehlermeldung, zu der auch ein Alarm zählt, wird daher von der Steuereirichtung nur veranlasst, wenn während der Verifizierungsphase kein Verwerfen-Kriterium erfüllt wird.

Die Verifizierungsphase kann eine vorbestimmte Verzögerungs-Zeitdauer oder eine vorbestimmte Verzögerungs-Anzahl an Atemhüben nach einer Erkennung eines Fehlerkandidaten beginnen. Bevorzugt beginnt die Verifizierungsphase mit dem Erkennen des Fehlerkandidaten. Dann, wenn die Verifizierungsphase als Anzahl von Atemhüben definiert ist, beginnt die Verifizierungsphase bevorzugt entweder unmittelbar mit dem Erkennen des Fehlerkandidaten oder mit einem Auslösen (Triggern) des auf den Atemhub, in welchem der Fehlerkandidat erkannt wurde, unmittelbar folgenden Atemhubs.

Die Genauigkeit, mit welcher ein einmal erkannter Fehlerkandidat wieder verworfen wird, kann noch dadurch erhöht werden, dass die Steuereinrichtung dazu ausgebildet ist, den Fehlerkandidaten zu verwerfen, wenn wenigstens ein Verwerfen-Kriterium am Ende der Verifizierungsphase erfüllt ist. Dann kann ein gleichsam falschpositives Verwerfen des Fehlerkandidaten aufgrund eines zufälligen und nur kurzen Erfüllens eines Verwerfen-Kriteriums verhindert werden. Dadurch kann sichergestellt sein, dass ab dem Erkennen eines Fehlerkandidaten eine Zeitspanne oder eine Anzahl an Atemhüben abgewartet wird, die üblicherweise erforderlich ist, um zu erkennen, ob eine Betriebssituation, die zur Identifikation eines Fehlerkandidaten führt, tatsächlich auf einer Störung des proximalen Durchflusssensors beruht oder eine andere Ursache hat.

Bevorzugt verwendet die Steuereinrichtung zur möglichst sicheren Vermeidung falsch-positiver Fehlermeldungen nicht nur ein Verwerfen-Kriterium, sondern eine Mehrzahl von Verwerfen-Kriterien. Dabei kann die Steuereinrichtung dazu ausgebildet sein, den Fehlerkandidaten nur dann zu verwerfen, wenn mehr als ein Verwerfen-Kriterium während der Verifizierungsphase erfüllt wird oder/und am Ende der Verifizierungsphase erfüllt ist.

In einer bevorzugten konkreten Ausführungsform kann das wenigstens eine Spontanatmung berücksichtigende Verwerfen-Kriterium umfassen:
- eine Anzahl von durch den mit der Beatmungsvorrichtung beatmeten Patienten ausgelösten aktiven Atemhüben des Patienten während der Verifizierungsphase übersteigt einen Aktiv-Atemhub-Schwellenwert.

Die Begriffe "Spontanatmung" und "aktiver Atemhub" sind in der vorliegenden Anmeldung insofern gleichbedeutend, als beide Begriffe einen durch den Patienten ausgelösten bzw. getriggerten Atemhub bezeichnen.

Grundsätzlich kann die Steuereinrichtung der Beatmungsvorrichtung mit einer weiteren Datenverarbeitungseinrichtung signalübertragungsmäßig gekoppelt sein und von der weiteren Datenverarbeitungseinrichtung Information über aktive Atemhübe des Patienten erhalten. Bevorzugt ist die Steuereinrichtung jedoch von weiteren Datenverarbeitungseinrichtungen möglichst unabhängig und daher dazu ausgebildet, einen aktiven Atemhub zu erkennen.

Grundsätzlich sind aus dem Stand der Technik zahlreiche Verfahren bekannt, aktive Atemhübe von mandatorischen Atemhüben automatisch zu unterscheiden und somit aktive Atemhübe auch während einer künstlichen Beatmung zu erkennen. Hierzu kann die Beatmungsvorrichtung unterschiedliche Sensoren nutzen, oder mit unterschiedlichen Sensoren signalübertragungsmäßig gekoppelt sein, um aus den von den Sensoren und gegebenenfalls von zwischenangeordneten Steuer- oder Auswerteeinrichtungen der Sensoren an die Steuereinrichtung übertragenen Daten und deren Auswertung einen aktiven Atemhub zu erkennen.

Im Fachbereich der künstlichen Beatmung sind unter dem Stichwort "assistierte Spontanatmung" bzw. "ASB" (= "Assisted Spontaneous Breathing") zahlreiche Arten und Weisen bekannt, vom Patienten ausgelöste aktive Atemhübe während einer künstlichen Beatmung zu erkennen. Diese können auch vorliegend Anwendung finden.

Besonders bevorzugt ist die Steuereinrichtung dazu ausgebildet, einen Zeitdauer-Erfassungswert zu erfassen, welcher eine Zeitdauer zwischen dem Auslösen eines Erfassungsatemhubs und dem Auslösen des dem Erfassungsatemhub unmittelbar nachfolgenden Atemhubs als Atemhubdauer des Erfassungsatemhubs repräsentiert. Die Steuereinrichtung ist weiter dazu ausgebildet, den Zeitdauer-Erfassungswert mit einem Betriebs-Zeitdauerwert zu vergleichen, welcher eine Zeitdauer zwischen dem jeweiligen mandatorischen Auslösen von zwei unmittelbar aufeinander folgenden durch die Beatmungsvorrichtung ausgelösten mandatorischen Atemhüben repräsentiert. Diese letztgenannte Zeitdauer ist eine mandatorische Atemhubdauer. Weiter ist die Steuereinrichtung dazu ausgebildet, den Erfassungsatemhub auf Grundlage des Zeitdauer-Vergleichsergebnisses als aktiven Atemhub zu erkennen. Mit "Erfassungsatemhub" ist dabei lediglich jener Atemhub bezeichnet, für welchen die Steuereinrichtung erkennen soll, ob es sich um einen aktiven oder einen mandatorischen Atemhub handelt.

Mit der vorgeschlagenen Erfassung eines aktiven Atemhubs auf Grundlage eines Vergleichs des Zeitdauer-Erfassungswerts mit dem Betriebs-Zeitdauerwert kann die Steuereinrichtung erkennen, ob der Erfassungsatemhub ein aktiver oder ein mandatorischer Atemhub ist, ohne dass hierfür über die bereits an der Beatmungsvorrichtung vorhandenen Sensoren hinaus weitere Sensoren notwendig sind. Da der vom Patienten getriggerte aktive Atemhub nur dann vom Patienten getriggert werden kann, wenn der Atemhub nicht bereits durch die Beatmungsvorrichtung getriggert wurde, weist ein unmittelbar vor einem aktiven Atemhub erfolgter Atemhub üblicherweise eine kürzere Atemhubdauer auf als die an der Beatmungsvorrichtung eingestellte mandatorische Atemhubdauer.

Anstelle der Atemhubdauer kann die zum Kehrwert der Atemhubdauer proportionale Atmungsfrequenz verwendet werden. Auch diese repräsentiert die Atemhubdauer eines Atemhubs.

Alternativ oder zusätzlich kann die Steuereinrichtung einen Atemhub als einen aktiven Atemhub erfassen bzw. erkennen, wenn sie dazu ausgebildet ist, einen Zeitdauer-Erfassungswert zu erfassen, welcher eine Zeitdauer zwischen dem Auslösen eines Erfassungsatemhubs und dem Auslösen des dem Erfassungsatemhub unmittelbar nachfolgenden Atemhubs als Atemhubdauer des Erfassungsatemhubs repräsentiert. Die Steuervorrichtung ist weiter dazu ausgebildet, den Erfassungswert mit einem Zeitdauer-Vergleichswert zu vergleichen, welcher eine über eine Mehrzahl von Atemhüben gemittelten Mittelwert von Atemhubdauern repräsentiert. Schließlich ist die Steuereinrichtung dazu ausgebildet, den Erfassungsatemhub auf Grundlage des Zeitdauer-Vergleichsergebnisses als aktiven Atemhub zu erkennen.

In der Regel folgt der Erfassungsatemhub der Mehrzahl von Atemhüben, über deren Atemhubdauern ein Mittelwert gebildet wird. Der Erfassungsatemhub kann auch Teil der Mehrzahl von Atemhüben sein, über deren Dauern der Mittelwert gebildet wird. Bevorzugt ist der Erfassungsatemhub dann der zeitlich letzte aus der Mehrzahl von Atemhüben.

Grundsätzlich kann jede Art von Mittelwertbildung angewendet werden. Bevorzugt wird ein arithmetischer Mittelwert gebildet. Dieser kann gewichtet sein oder auch nicht.

Klinische Versuche haben gezeigt, dass die Atemhubdauern bei einsetzender Spontanatmung kürzer sind als die Atemhubdauern mandatorischer Atemhübe, und dass insbesondere nacheinander folgende aktive Atemhübe zunehmend kürzere Atemhubdauern aufweisen. Kürzer werdenden Atemhubdauern stehen größer werdende Atemfrequenzen gleich.

Weiter ist es möglich, dass die Mehrzahl von Atemhüben, welche zur Mittelwertbildung von Atemhubdauern herangezogen werden, für die Beurteilung mehrerer unterschiedlicher Erfassungsatemhübe stets die gleiche Mehrzahl von Atemhüben ist. Die zum Vergleich herangezogenen Atemhübe können dabei stets dieselben Atemhübe sein, so dass der Mittelwert nur einmal oder nur in großen Zeitabständen berechnet zu werden braucht.

Eine möglichst genaue, wenig fehlerbehaftete Erkennung aktiver Atemhübe kann dadurch erfolgen, dass der Zeitdauer-Vergleichswert einen gleitenden Mittelwert von Atemhubdauern über eine vorbestimmte Anzahl von bis zum Erfassungsatemhub aufeinander folgenden Atemhüben wiedergibt. Wiederum kann der Erfassungsatemhub auf den letzten Atemhub der zur gleitenden Mittelwertbildung herangezogenen Mehrzahl von Atemhüben folgen oder kann Teil der Mehrzahl von Atemhüben sein. Bevorzugt ist er der zeitlich letzte aus der Mehrzahl von Atemhüben. Der gleitende Mittelwert ist bevorzugt ein arithmetischer Mittelwert, der gewichtet sein kann, aber nicht gewichtet sein muss.

Bevorzugt wird ein gleitender Mittelwert oder überhaupt ein Mittelwert von Atemhubdauern über eine Anzahl von fünf bis zehn Atemhüben, besonders bevorzugt von acht Atemhüben, gebildet. Besonders bevorzugt folgen die zur Mittelwertbildung von Atemhubdauern herangezogenen Atemhübe zeitlich unmittelbar aufeinander.

Zusätzlich oder alternativ zur Heranziehung einer Atemhubdauer oder einer Atemhubfrequenz zur Erkennung von aktiven Atemhüben kann die Steuereinrichtung der Beatmungsvorrichtung ohne zusätzliche Sensorik einen aktiven Atemhub auch anhand der ohnehin durch die Drucksensoranordnung vorhandenen Druckwerte des Beatmungsgasdrucks von in der Beatmungsleitungsanordnung strömenden Beatmungsgas erkennen. Dies liegt technisch darin begründet, dass in der Regel bei einem ersten aktiven Atemhub ein höherer Beatmungsgasfluss erfasst wird als bei einem mandatorischen Atemhub, da zur mandatorischen Beatmung die Aktivität des Patienten hinzutritt. Als Folge davon wird bei der hier betrachteten Druckregelung zur Erzielung eines Soll-Flusses bzw. eines Soll-Tidalvolumens der Beatmungsgasdruck des nachfolgenden Atemhubs abgesenkt. Somit kann die Steuereinrichtung der Beatmungsvorrichtung auch dadurch einen Erfassungsatemhub als aktiven Atemhub erkennen, dass die Steuereinrichtung dazu ausgebildet ist, einen Gasdruck von in der Beatmungsleitungsanordnung strömendem Gas zu erfassen und einen während eines Erfassungsatemhubs erfassten Gasdruck mit dem während eines vorhergehenden Atemhubs erfassten Gasdruck zu vergleichen, wobei die Steuereinrichtung dazu ausgebildet ist, den Erfassungsatemhub auf Grundlage des Druck-Vergleichsergebnisses als aktiven Atemhub zu erkennen. Bevorzugt ist der erfasste Gasdruck ein Druck eines inspiratorischen Beatmungsgases.

Der vorhergehende Atemhub ist bevorzugt ein unmittelbar dem Erfassungsatemhub vorhergehender Atemhub, wobei nicht ausgeschlossen sein soll, dass zwischen dem vorhergehenden Atemhub und dem Erfassungsatemhub wenigstens ein weiterer Atemhub gelegen ist.

Zur Erhöhung der Genauigkeit der Erkennung eines aktiven Atemhubs ist die Steuereinrichtung der Beatmungsvorrichtung bevorzugt dazu ausgebildet, sowohl einen Zeitdauer-Vergleich wie auch einen Druck-Vergleich auszuführen, wobei der Erfassungsatemhub dann auf Grundlage sowohl des Zeitdauer-Vergleichsergebnisses als auch des Druck-Vergleichsergebnisses als aktiver Atemhub erkannt wird oder nicht.

Zusätzlich oder alternativ kann die Steuereinrichtung der Beatmungsvorrichtung dazu ausgebildet sein, während eines Erfassungsatemhubs wenigstens einen Wert eines Ösophagus-Drucks oder/und eines Pleuraldrucks des Patienten zu erfassen und auf Grundlage des wenigstens einen erfassten Druckwerts den Erfassungsatemhub als aktiven Atemhub zu erkennen, wobei hierfür über die eingangs als Bestandteil der Beatmungsvorrichtung genannten Sensoranordnungen weitere Sensoren nötig sind. Der Pleuraldruck wird häufig auch als "Interpleuraldruck" bezeichnet.

Die vom Patienten während einer künstlichen Beatmung gezeigte Spontanatmung ist jedoch in der Regel nur dann für eine falsch-positive Fehlerkandidatenerkennung relevant, wenn während der Verifizierungsphase ausreichend viele aktive Atemhübe aufgetreten sind. Daher kann der Aktiv-Atemhub-Schwellenwert, dessen Überschreiten als Verwerfen-Kriterium gilt, als vorbestimmter Aktiv-Atemhub-Schwellenwert wenigstens 39 % der Gesamtanzahl an Atemhüben in der Verifizierungsphase betragen.

Der Aktiv-Atemhub-Schwellenwert kann wiederum während der künstlichen Beatmung des Patienten oder sogar während der Verifizierungsphase auf Grundlage von Betriebsdaten der Beatmungsvorrichtung oder/und aufgrund von Patientendaten individuell bestimmt werden oder kann als vorbestimmter Schwellenwert in einem Speicher hinterlegt sein. Bevorzugt ist der Aktiv-Atemhub-Schwellenwert ein vorbestimmter Schwellenwert.

Da die Spontanatmung während der Verifizierungsphase einen umso größeren Einfluss auf die Fehlerkandidaten-Erkennung durch die Steuereinrichtung hat, je mehr aktive Atemhübe durch den Patienten selbst statt durch die Beatmungsvorrichtung ausgelöst bzw. getriggert werden, kann der Aktiv-Atemhub-Schwellenwert in einer weiter bevorzugten Ausführungsform der Beatmungsvorrichtung vorzugsweise wenigstens 45 %, noch stärker bevorzugt wenigstens 50 % der Gesamtanzahl an Atemhüben in der Verifizierungsphase betragen.

Dann jedoch, wenn zwar Spontanatmung in der Verifizierungsphase erkannt wird, jedoch der Aktiv-Atemhub-Schwellenwert nicht erreicht wird, kann eine gewisse Unsicherheit in der Bewertung der von der Steuereinrichtung erfassten vorrichtungsspezifischen oder/und patientenspezifischen Parameter eintreten. Um diese Unsicherheit zu beseitigen oder wenigstens zu verringern, kann die Steuereinrichtung dazu ausgebildet ist, dann wenn die während der vorbestimmten Verifizierungsphase ermittelte Anzahl an aktiven Atemhüben größer als Null und kleiner als der vorbestimmte Aktiv-Atemhub-Schwellenwert ist, die Verifizierungsphase um eine Zusatz-Verifizierungsphase zu verlängern.

Die Zusatz-Verifizierungsphase kann ausgehend von individuell vorliegenden Betriebs- oder/und Patientendaten ermittelt werden oder kann eine vorbestimmte Zusatz-Verifizierungsphase sein, die in einem mit der Steuereinrichtung zusammenwirkenden Datenspeicher hinterlegt ist. Die Dauer der verlängerten Verifizierungsphase beträgt dann die Dauer der ursprünglichen Verifizierungsphase plus die Dauer der Zusatz-Verifizierungsphase.

Bevorzugt wendet die Steuereinrichtung auch dann, wenn die Verifizierungsphase um die Zusatz-Verifizierungsphase verlängert ist, das gleiche wenigstens eine Verwerfen-Kriterium an wie in der ursprünglichen Verifizierungsphase, d. h. es wird lediglich die Verifizierungsphase verlängert, eine Anpassung des wenigstens einen Verwerfen-Kriteriums erfolgt mit der Verlängerung der Verifizierungsphase nicht.

Grundsätzlich kann das oben beschriebene, eine Spontanatmung des Patienten berücksichtigende Verwerfen-Kriterium das einzige Verwerfen-Kriterium sein, dessen Erfüllung zum Verwerfen des Fehlerkandidaten und damit zur Beurteilung eines möglicherweise kritischen Betriebszustands der Beatmungsvorrichtung, insbesondere des proximalen Durchflusssensors, als nicht-fehlerbehaftet führt. Die Genauigkeit der Erkennung eines Fehlers der Beatmungsvorrichtung, insbesondere des proximalen Durchflusssensors, kann jedoch noch dadurch weiter erhöht werden, dass das wenigstens eine Verwerfen-Kriterium zusätzlich umfasst:
- ein betragsmäßiger Volumen-Unterschiedswert, welcher einen Unterschied zwischen dem mittels dem distalen Durchflusssensor ermittelten distalen Gasvolumen und dem mittels dem proximalen Durchflusssensor während desselben Atemhubs ermittelten proximalen Gasvolumens angibt, liegt innerhalb eines Volumen-Unterschiedswerte-Zulässigkeitsbereichs, oder/und
- ein betragsmäßiger Druck-Unterschiedswert, welcher einen Unterschied zwischen den während unterschiedlicher Atemhübe von der Drucksensoranordnung erfassten Gasdrücken angibt, liegt innerhalb eines Druck-Unterschiedswerte-Zulässigkeitsbereichs.

Wiederum kann einer oder beide der Zulässigkeitsbereiche vorbestimmt in einem mit der Steuereinrichtung zusammenwirkenden Datenspeicher hinterlegt sein oder können individuell abhängig von Betriebs- oder/und Patientendaten zum Zeitpunkt der Erkennung des Fehlerkandidaten ermittelt werden.

Üblicherweise wird der Fehlerkandidat nämlich dadurch erkannt, dass der betragsmäßige Volumen-Unterschiedswert außerhalb eines Volumen-Unterschiedswerte-Zulässigkeitsbereichs liegt oder/und dass der betragsmäßige Druck-Unterschiedswert außerhalb eines Druck-Unterschiedswerte-Zulässigkeitsbereichs liegt. Insofern kann als Verwerfen-Kriterium auch dienen, dass die erfüllte Bedingung zur Erkennung eines Fehlerkandidaten während der Verifizierungsphase aufhört vorzuliegen oder wenigstens am Ende der Verifizierungsphase nicht mehr vorliegt.

Bevorzugt ist die Steuereinrichtung derart ausgebildet, dass sie den Fehlerkandidaten sofort verwirft, wenn während der Verifizierungsphase - das schließt die oben genannte verlängerte Verifizierungsphase ein - einer oder beide Unterschiedswerte innerhalb des jeweils zugeordneten Unterschiedswerte-Zulässigkeitsbereichs liegt, und dass der Fehlerkandidat erst dann verworfen wird, wenn am Ende der Verifizierungsphase - dies schließt die verlängerte Verifizierungsphase ein - die Gesamtanzahl an Atemhüben über dem Aktiv-Atemhub-Schwellenwert liegt.

Die Steuereinrichtung der in der vorliegenden Anmeldung vorgeschlagenen Beatmungsvorrichtung ist gemäß einer vorteilhaften Weiterbildung der vorliegenden Erfindung konkret derart zur Steuerung des Betriebs der Druckveränderungsanordnung auf Grundlage von Messsignalen des proximalen Durchflusssensors ausgebildet, dass sie im bestimmungsgemäßen mandatorischen Beatmungsbetrieb die Druckveränderungsanordnung zur Veränderung des Gasdrucks des in der Beatmungsleitungsanordnung strömenden inspiratorischen Beatmungsgases nach Maßgabe des Messsignals des proximalen Durchflusssensors derart ansteuert, dass ein auf Grundlage des vom proximalen Durchflusssensor erfassten Gasflusswerts ermittelter Gasvolumenwert innerhalb eines vorgegebenen Volumen-Wertebereichs liegt, vorzugsweise im Wesentlichen konstant ist.

Dies betrifft den von der Anmelderin bekannten Beatmungs-Betriebsmodus "APV", welcher oben bereits erläutert wurde. Der Gasvolumenwert kann beispielsweise aus dem einen Gasfluss repräsentierenden Messsignal des proximalen Durchflusssensors mittels Integration über die Zeit ermittelt werden.

Grundsätzlich kann dabei ein proximaler Durchflusssensor eingesetzt werden, welcher nach einem beliebigen physikalischen Wirkprinzip arbeitet, solange er nur in der Lage ist, den Beatmungsgasfluss am Ort seiner Anordnung zu messen. Beispielsweise kann der proximale Durchflusssensor ein Heißdrahtanemometer umfassen. Bevorzugt ist der proximale Durchflusssensor jedoch ein Differenzdrucksensor, wobei die Drucksensoranordnung wenigstens einen der am Differenzdrucksensor erfassten Gasdrücke als den Gasdruck von in der Beatmungsleitungsanordnung strömendem Gas erfasst und dass die Steuereinrichtung diesen erfassten Gasdruck zur Ermittlung eines Fehlerkandidaten des proximalen Durchflusssensors verwendet. Denn so kann der proximale Durchflusssensor auch zur Druckerfassung von Beatmungsgasdruck in der Beatmungsleitungsanordnung dienen.

Um den Patienten vor einer fehlerhaften Beatmung zu schützen, ist die Steuereinrichtung der Beatmungsvorrichtung vorzugsweise dazu ausgebildet, dann, wenn sie auf einen Fehler in dem proximalen Durchflusssensor schließt, einen Steuereingriff an der Beatmungsvorrichtung vorzunehmen. Ein derartiger Steuereingriff kann die Ausgabe einer Fehlermeldung, einschließlich eines Alarms, sein. Dies kann auch fernab des Aufstellungsorts der Beatmungsvorrichtung erfolgen, etwa durch Funksignale an einem Ort, in dem sich Pflegepersonal aufhält oder bereithält. Ebenso kann die Steuereinrichtung dazu ausgebildet sein, nach Erkennung des Fehlers die künstliche Beatmung des Patienten mit Betriebsparametern fortzusetzen, die in einem vorbestimmten Zeitpunkt oder in einem vorbestimmten Zeitraum vor Erkennung des Fehlerkandidaten verwendet wurden. Ein solcher hilfsweiser Beatmungsbetrieb kann selbstverständlich nur über kurze Zeit aufrechterhalten werden. Jedoch kann so ermöglicht werden, dass der Patient mit zutreffenderen Betriebsparametern beatmet wird als auf Grundlage von jenen, die aktuell durch den fehlerhaften proximalen Durchflusssensor geliefert werden. Mit gleichzeitiger Ausgabe einer Fehlermeldung oder eines Alarms ist die Zeit für einen solchen Notbetrieb begrenzt.

Die vorliegende Erfindung wird nachfolgend anhand der beiliegenden Zeichnungen näher erläutert. Es stellt dar:
- Figur 1: eine Ausführungsform einer erfindungsgemäßen Beatmungsvorrichtung,
- Figur 2: grobschematische Kurven des in eine Patientenlunge verbrachten Beatmungsgasvolumens und des Beatmungsgasdrucks in der Beatmungsleitungsanordnung als Funktionen der Zeit bei rein mandatorischer Beatmung,
- Figur 3: grobschematische Kurven des Beatmungsgasvolumens, des Beatmungsgasdrucks und eines Ösophagus-Drucks als Funktion der Zeit bei assistierter Spontanatmung,
- Figur 4: ein beispielhafter klinischer Kurvenverlauf der Atemfrequenz des Patienten, des distalen Beatmungsgasvolumens, des proximalen Beatmungsgasvolumens und des Beatmungsgasdrucks, aufgetragen über die Zeit, bei erkanntem, aber verworfenem Fehlerkandidaten,
- Figur 5: ein Diagramm derselben Parameter wie in Figur 4, aufgetragen über die Zeit, mit erkanntem, aber aus anderem Grunde wie im Diagramm gemäß Figur 4 verworfenem Fehlerkandidaten, und
- Figur 6: ein drittes grobschematisches Diagramm mit den Parametern von Figur 5 mit Erkennung eines Fehlerkandidaten und Erkennung eines Fehlers daraus.

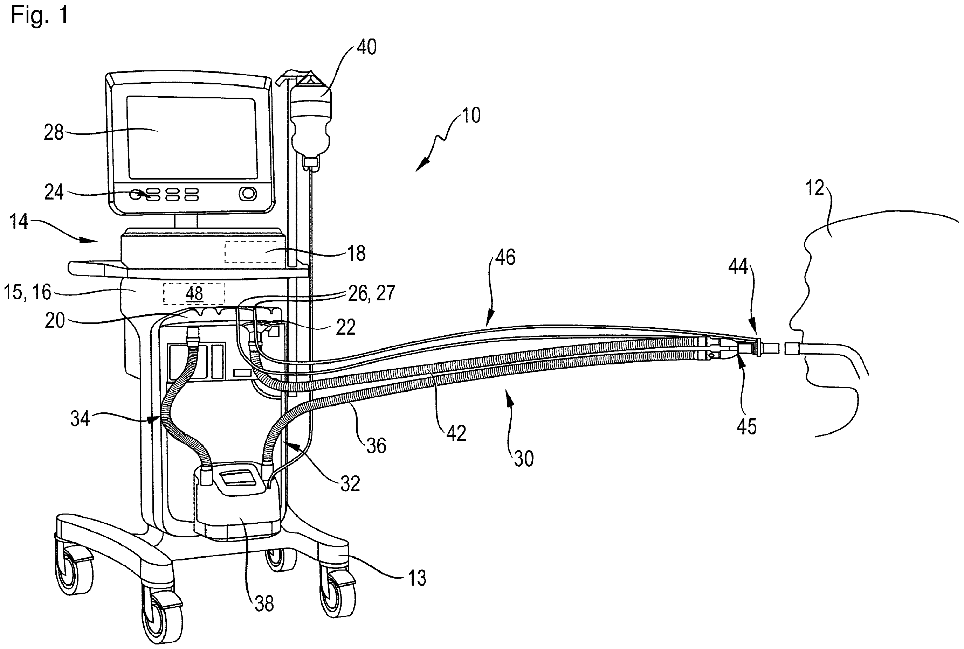

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 13 aufgenommen sein kann.

Die Beatmungsvorrichtung 10 weist ein Gehäuse 14 auf, in dem - von außen wegen des blickdichten Gehäusematerials nicht erkennbar - eine Druckveränderungsanordnung 16 und eine Steuereinrichtung 18 aufgenommen sein können.

Die Druckveränderungsanordnung 16 ist in an sich bekannter Weise aufgebaut und weist eine Beatmungsgasquelle 15 in Gestalt einer Pumpe, eines Verdichters oder eines Gebläses auf, die jeweils lastveränderlich ansteuerbar sind und daher nicht nur der Einleitung von Beatmungsgas in die Beatmungsvorrichtung, sondern auch der Änderung des Drucks des eingeleiteten Beatmungsgases dienen. Die Beatmungsgasquelle 15 (Gasquelle 15) kann alternativ auch durch einen Druckbehälter gebildet sein, welcher an das Gehäuse 14 der Beatmungsvorrichtung 10 anschließbar ist. Die Druckveränderungsanordnung 16 kann die Gasquelle 15 und gegebenenfalls zusätzlich - oder im Falle eines unter Druck stehenden Gasvorrats als Gasquelle alternativ - ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst eine in Figur 1 nicht dargestellte Speichereinrichtung, um für den Betrieb der Beatmungsvorrichtung 10 notwendige Daten speichern und erforderlichenfalls aufrufen zu können. Die Speichereinrichtung kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Speichereinrichtung bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 weist die Beatmungsvorrichtung 10 einen Dateneingang 24 auf, welcher in dem in Figur 1 dargestellten Beispiel durch eine Tastatur repräsentiert ist. Die Steuereinrichtung 18 kann alternativ oder zusätzlich zur dargestellten Tastatur Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Druckveränderungsanordnung 16 im Gehäuse 14, über eine Beatmungsleitungsanordnung 30 verbunden. Der Patient 12 ist hierfür intubiert.

Die Beatmungsleitungsanordnung 30, über welche frisches Beatmungsgas von der Gasquelle 15 und der Druckveränderungsanordnung 16 in die Lunge des Patienten 12 geleitet werden kann, weist außerhalb des Gehäuses 14 einen Inspirationsschlauch 32 auf. Der Inspirationsschlauch 32 kann unterbrochen sein und einen ersten Inspirationsteilschlauch 34 und einen zweiten Inspirationsteilschlauch 36 aufweisen, zwischen welchen eine Konditionierungseinrichtung 38 zur gezielten Befeuchtung und gegebenenfalls auch Temperierung des dem Patienten 12 zugeführten frischen Beatmungsgases vorgesehen sein kann. Die Konditionierungseinrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, dem Beatmungsgas zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Konditionierungseinrichtung 38 sorgt dafür, dass das frische Beatmungsgas dem Patienten 12 mit einem vorbestimmten Feuchtegehalt, gegebenenfalls unter Zugabe eines Medikamenten-Aerosols, und mit einer vorbestimmten Temperatur zugeleitet wird.

Die Beatmungsleitungsanordnung 30 weist neben dem bereits erwähnten Inspirationsventil 20 das Exspirationsventil 22 und weiter einen Exspirationsschlauch 42 auf, über welchen verstoffwechseltes Beatmungsgas aus der Lunge des Patienten 12 in die Atmosphäre abgeblasen wird.

Der Inspirationsschlauch 32 ist mit dem Inspirationsventil 20 gekoppelt, der Exspirationsschlauch 42 mit dem Exspirationsventil 22. Von den beiden Ventilen ist jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches Beatmungsgas vom Gehäuse 14 zum Patienten 12 geleitet werden kann. Eine Strömung des frischen Beatmungsgases wird durch gezielte Druckerhöhung des Beatmungsgases durch die Druckveränderungsanordnung 16 bewirkt. Aufgrund der Druckerhöhung strömt das frische Beatmungsgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Beatmungsgases strömt dieses nach dem Öffnen des Exspirationsventils 22 in die Atmosphäre, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck, also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Beatmungsgasvolumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu ist die Beatmungsvorrichtung 10 mit einem oder mehreren Sensoren daten-übertragungsmäßig verbunden, welche den Zustand des Patienten oder/und den Betrieb der Beatmungsvorrichtung überwachen.

Einer dieser Sensoren ist ein proximaler Durchflusssensor 44, welcher in einem Y-Verbindungsstück 45 die dort in der Beatmungsleitungsanordnung 30 herrschende Beatmungsgas-Strömung erfasst. Der Durchflusssensor 44 kann mittels einer Sensor-Leitungsanordnung 46 mit den Dateneingängen 26 der Steuereinrichtung 18 gekoppelt sein. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Durchflusssensors 44 herrschenden Gasdruck an die Dateneingänge 26 übertragen, wo diese von Drucksensoren 27 quantifiziert werden. Der Durchflusssensor 44 ist vorliegend als Differenzdruck-Durchflusssensor 44 dargestellt. Der Durchflusssensor 44 ist zwar bevorzugt ein nach dem Differenzdruck-Prinzip arbeitender Durchflusssensor, kann jedoch auch ein nach einem anderen physikalischen Wirkprinzip arbeitender Durchflusssensor sein.

Im Gehäuse 14 ist ein weiterer Durchflusssensor 48 vorgesehen, welcher aufgrund seiner größeren Entfernung vom Patienten 12 - verglichen mit dem proximalen Durchflusssensor 44 - als distaler Durchflusssensor 48 bezeichnet ist.

Die Messsignale der Drucksensoren 27 können zur Durchführung eines besonders vorteilhaften Betriebsmodus verwendet werden, welcher in der Fachwelt als "Adaptive Pressure Ventilation" oder kurz APV bekannt sind. Dabei wird - vereinfacht ausgedrückt - das aus dem vom proximalen Durchflusssensor 44 gemessenen Beatmungsgasfluss ermittelte Beatmungsgasvolumen durch Veränderung des Drucks des Beatmungsgases in der Beatmungsleitungsanordnung 30 durch die Druckveränderungsanordnung 16 so verändert, dass das mittels des proximalen Durchflusssensors 44 ermittelte Beatmungsgasvolumen einem vorgegebenen Sollwert entspricht oder in einem vorgegebenen Sollwertebereich gelegen ist. Der Sollwert bzw. Sollwertebereich kann dabei durch einen behandelnden Arzt über die Eingabevorrichtung vorgegeben werden oder kann aus den der Steuereinrichtung 18 zugänglichen Patientendaten errechnet werden.

Im Betriebsmodus "APV" führt somit eine Änderung des mittels des proximalen Durchflusssensors 44 ermittelten Beatmungsgasvolumens in der Regel zu einer Änderung des Drucks des Beatmungsgases in der Beatmungsleitungsanordnung 30.

Aufgrund des Ortes seiner Anbringung in dem Y-Verbindungsstück 45 ist der proximale Durchflusssensor 44 im Gegensatz zum distalen Durchflusssensor 48 grundsätzlich auch in der Lage, den Fluss von exspiratorischem Beatmungsgas durch den Exspirationsschlauch 42 zu erfassen.

Die korrekte Funktion der Durchflusssensoren 44 und 48 ist für den korrekten Betrieb der Beatmungsvorrichtung 10 und somit für die Gesundheit des Patienten 12 wesentlich.

Es hat sich im Betrieb gezeigt, dass gerade der proximale Durchflusssensor 44 aufgrund seiner Nähe zum Patienten 12 einem größeren Fehlerrisiko unterliegt als der distale Durchflusssensor 48. Der proximale Durchflusssensor 44, durch den auch exspiratorisches Beatmungsgas strömt, ist beispielsweise stärker als der distale Durchflusssensor 48 durch im Beatmungsgas enthaltene Feuchtigkeit belastet. Dies gilt umso mehr, wenn der distale Durchflusssensor 48 wie im vorliegenden Beispiel der Figur 1 in Inspirationsrichtung stromaufwärts der Konditionierungseinrichtung 38 angeordnet ist und somit im Wesentlichen nur von trockenem inspiratorischem Beatmungsgas durchströmt wird.

Die Steuereinrichtung 18 der erfindungsgemäßen Beatmungsvorrichtung 10 ist zur Überwachung des Betriebs der aus dem proximalen Durchflusssensor 44 und dem distalen Durchflusssensor 48 gebildeten Durchflusssensoranordnung ausgebildet, um eine Fehlfunktion der Durchflusssensoranordnung rechtzeitig erkennen zu können.

Üblicherweise misst der distale Durchflusssensor 48 einen betragsmäßig größeren Beatmungsgasfluss, was zu einem daraus ermittelten betragsmäßig größeren Beatmungsgasvolumen führt, als der proximale Durchflusssensor 44, da der distale Durchflusssensor 48 näher an der Beatmungsgasquelle 15 gelegen ist als der proximale Durchflusssensor 44 und damit weniger gasflussmindernden Fehlereinflüssen unterliegt.

Ein solcher Einfluss ist beispielsweise die Elastizität der Beatmungsleitungsanordnung 30, insbesondere des Inspirationsschlauchs 32. Wird in diesen inspiratorisches Beatmungsgas eingeleitet, welches systemimmanent gegenüber der umgebenden Atmosphäre unter Überdruck stehen muss, verrichtet das eingeleitete inspiratorische Beatmungsgas Arbeit gegen die Elastizität des Inspirationsschlauchs 32 und dehnt diesen. In dem durch diese Dehnung vergrößerten Volumen des Inspirationsschlauchs 32 wird Beatmungsgas aufgenommen, welches zwar den distalen Durchflussmesser 48 durchströmt hat, jedoch den Patienten 12 und den unmittelbar vor ihm liegenden proximalen Durchflusssensor 44 nicht mehr erreicht.

Darüber hinaus schließt das Exspirationsventil 22 in vielen Fällen nicht hermetisch dicht ab, so dass in der Inspirationsphase ein Querstrom in strömungsmechanischem Kurzschluss zwischen Inspirationsventil 20 und Exspirationsventil 22 entsteht. In diesem Querstrom strömt zwar Beatmungsgas, das den distalen Durchflusssensor 48 durchströmt, jedoch weder den proximalen Durchflusssensor 44, noch den Patienten 12 erreicht, sondern zuvor in dem Y-Verbindungsstück 45 unmittelbar vom Inspirationsschlauch 32 in den Exspirationsschlauch 42 strömt.

Daher liegt das Niveau der Flusserfassung und damit verbunden der Volumenermittlung mittels des distalen Durchflusssensors 48 betragsmäßig höher als das Niveau der Flusserfassung und damit verbunden der Volumenermittlung mittels des proximalen Durchflusssensors 44.

Wie oben bereits angedeutet wurde, kann sich Feuchtigkeit aus dem Beatmungsgas im proximalen Durchflusssensor 44 niederschlagen und dessen Messergebnis verfälschen. Die Folge eines flüssigkeitsbelasteten proximalen Durchflusssensors 44 ist nach bisheriger Erfahrung stets ein Messsignal, welches einen betragsmäßig höheren Beatmungsgasfluss anzeigt als tatsächlich vorliegt. Dies führt in der Folge zu einem daraus ermittelten Beatmungsgasvolumen, das einen betragsmäßig höheren Wert aufweist als das tatsächlich verabreichte.

Ein übermäßig von Flüssigkeit belasteter und daher fehlerhaft erfassender proximaler Durchflusssensor 44 regeneriert von selbst nicht mehr und muss gegen einen fehlerfreien, von Flüssigkeit unbelasteten Sensor ausgetauscht werden, um eine korrekte Beatmung des Patienten 12 weiterhin sicherzustellen.

Ein ähnlicher Effekt, also ein erhöhter proximaler Durchfluss und in der Folge ein erhöhtes proximales Beatmungsgasvolumen, kann jedoch auch aufgrund anderer Ursachen als einer übermäßigen Flüssigkeitsbelastung des proximalen Durchflusssensors 44 eintreten. So kann auch während der künstlichen Beatmung auftretende Spontanatmung den betragsmäßigen Unterschiedswert zwischen dem vom distalen Durchflusssensor 48 gemessenen distalen Beatmungsgasfluss sowie dem daraus ermittelten distalen Beatmungsgasvolumen und dem vom proximalen Durchflusssensor 44 gemessenen proximalen Beatmungsgasfluss sowie dem daraus ermittelten proximalen Beatmungsgasvolumen bezogen auf den Unterschiedswert bei rein mandatorischer Atmung verändern.

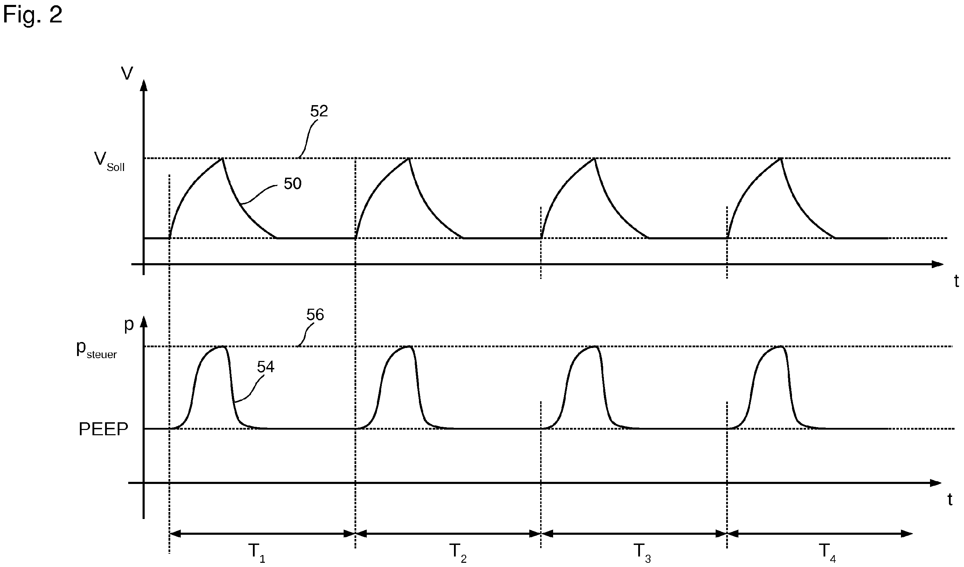

Figur 2 zeigt - lediglich grobschematisch zur Veranschaulichung - in einem oberen Diagramm das dem künstlich beatmeten Patienten 12 mandatorisch zugeführte Beatmungsgasvolumen als Funktion der Zeit, wobei die Kurve des Beatmungsgasvolumens als Funktion der Zeit mit dem Bezugszeichen 50 bezeichnet ist. Eine Soll-Vorgabe des Tidalvolumens ist mit der waagerechten gestrichelten Linie 52 dargestellt. In Figur 2 sind vier zeitlich aufeinanderfolgende Atemhübe aufgetragen. Unter dem Beatmungsgasvolumen als Funktion der Zeit ist mit gleicher Zeitskala und ebenfalls nur grobschematisch der Beatmungsgasdruck als Funktion der Zeit in der Kurve 54 dargestellt. Ein von der Steuereinrichtung 18 der Druckveränderungsanordnung 16 vorgegebener maximaler Steuerdruck ist mit Bezugszeichen 56 bezeichnet. Er ist in der in Figur 2 dargestellten rein mandatorischen künstlichen Beatmung über die dargestellten vier Atemhübe hinweg konstant. Aus an sich bekannten Gründen steuert die Steuereinrichtung 18 die Druckveränderungsanordnung 16 derart, dass der Beatmungsgasdruck in der Beatmungsleitungsanordnung nicht unter den positiven end-exspiratorischen Druck "PEEP" fällt.

Dabei ist sichergestellt, dass dann, wenn der Beatmungsgasdruck in der Beatmungsleitungsanordnung 30 den von der Steuereinrichtung 18 vorgegebenen Steuerdruck psteuer erreicht, das dem Patienten zugeführte Tidalvolumen dem Soll-Tidalvolumen V_{Soll} entspricht. Das Tidalvolumen gemäß der grobschematischen Kurve 50 ist mit dem proximalen Durchflusssensor 44 ermittelt worden. Dann, wenn in einem Atemhub das dem Patienten zugeführte Beatmungsgasvolumen nicht dem vorgegebenen Sollvolumen V_{Soll} ausreichend entspricht, wird von der Steuereinrichtung 18 für den nachfolgenden Atemhub ein erhöhter oder verringerter Steuerdruck psteuer vorgegeben, um so das dem Patienten 12 zugeführte Beatmungsgasvolumen für jeden Atemzug so konstant wie möglich zu halten.

Ein Atemhub dauert dabei von dem Zeitpunkt an, an welchem sich die Kurve 50 des Beatmungsgasvolumens als Funktion der Zeit ausgehend von ihrer Grundlinie zu höheren Werten hin verändert. Die Phase größer werdender Volumenwerte entspricht einer Inspirationsphase, die Phase abnehmender Volumenwerte entspricht einer Exspirationsphase. Der Zeitraum zwischen dem Beginn zweier zeitlich unmittelbar aufeinanderfolgender Inspirationsphasen ist die Atemhubdauer Tᵢ mit i = 1 bis 4 in Figur 2.

Da die in Figur 2 dargestellte künstliche Beatmung rein mandatorisch, also ausschließlich durch die Beatmungsvorrichtung 10 ausgelöst bzw. getriggert erfolgt, sind alle in Figur 2 gezeigten Atemhubdauern T₁ bis T₄ gleich lang.

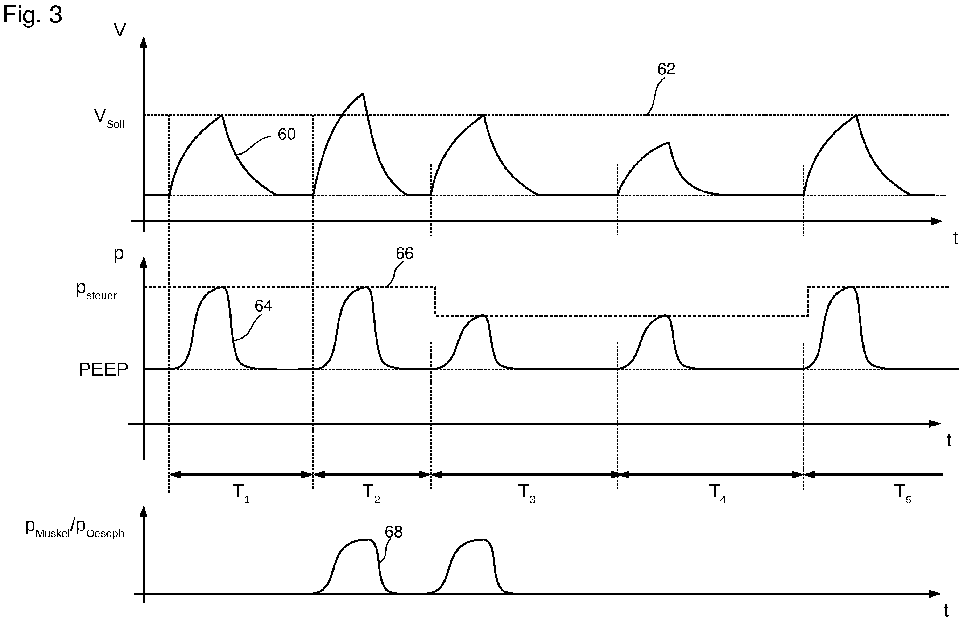

In Figur 3 sind die gleichen Datenkurven wie in Figur 2 dargestellt, jedoch bei auftretender Spontanatmung. Gleiche Graphen bzw. Größen wie in Figur 2 sind in Figur 3 mit gleichen Bezugszeichen versehen, jedoch erhöht um die Zahl 10.

Figur 3 zeigt fünf zeitlich unmittelbar aufeinanderfolgende Atemhübe bzw. die zu diesen Atemhüben gehörenden Beatmungsgasvolumina als Funktion der Zeit und die Beatmungsgasdrücke in der Beatmungsleitungsanordnung 30 als Funktion der Zeit.

Der erste Atemhub in Figur 3 ist ein mandatorischer Atemhub. Er entspricht mit Ausnahme seiner unten näher erläuterten verkürzten Atemhubdauer dem ersten Atemhub in Figur 2.

In Figur 3 ist der zweite Atemhub ein aktiver Atemhub, welcher durch den Patienten ausgelöst wurde. Da der Patient 12 zusätzlich zu dem ihm über die Beatmungsvorrichtung 10 zugeführten Beatmungsgas selbstständig Beatmungsgas ansaugt, erhält er im zweiten Atemhub insgesamt ein größeres Beatmungsgasvolumen als von der Steuereinrichtung 18 der Beatmungsvorrichtung 10 vorgesehen ist. Dies erfasst der proximale Durchflusssensor 44. Die Steuereinrichtung 18 senkt daher den Steuerdruck psteuer des Beatmungsgases für den nachfolgenden dritten Atemhub, um das dem Patienten während eines Atemhubs zugeführte Beatmungsgasvolumen wieder auf seinen Sollwert V_{Soll} (siehe Soll-Kurve 62 in Figur 3) zurückzuführen. Da auch der dritte Atemhub von Figur 3 ein aktiver Atemhub ist, gelingt die Rückführung des dem Patienten 12 zugeführten Beatmungsgasvolumens auf das vorgegebene Soll-Niveau.

Da der Patient 12 den dritten Atemhub triggert, ist die Atemhubdauer T₂ des zweiten Atemhubs gegenüber der vorgegebenen Atemhubdauer eines mandatorischen Atemhubs verkürzt. Gleiches gilt im auch für die Atemhubdauer T₁ des ersten Atemhubs, wenngleich dies in Figur 3 nicht so deutlich ausfällt wie für den zweiten Atemhub.

Der vierte Atemhub ist wieder ein rein mandatorischer Atemhub, der mit dem bereits für den dritten Atemhub geltenden Steuerdruck psteuer ausgeführt wird, der für den dritten Atemhub zu der gewünschten Zuführung des Soll-Beatmungsgasvolumens geführt hat. Da im vierten Atemhub die Spontanatmung des Patienten ausbleibt, ist der maximal erreichte Druck psteuer des Beatmungsgases während der Inspirationsphase jedoch zu gering, um das Soll-Beatmungsgasvolumen V_{Soll} dem Patienten zuzuführen. Dementsprechend ist das dem Patienten 12 im vierten Atemhub zugeführte Beatmungsgasvolumen zu gering, woraufhin die Steuereinrichtung 18 für den nachfolgenden fünften Atemhub die Druckveränderungsanordnung 16 zur Bereitstellung eines höheren maximalen Beatmungsgasdrucks psteuer ansteuert.

Die Beatmungsdauern T₁, T₂ und T₃ sind aufgrund der Spontanatmung in den Atemhüben 2 und 3 unterschiedlich lang. Ein einem aktiven Atemhub unmittelbar vorausgehender rein mandatorischer Atemhub ist zeitlich üblicherweise gegenüber einem rein mandatorischen Atemhub, dem ein weiterer rein mandatorischer Atemhub folgt, verkürzt. Ein einem aktiven Atemhub bei der gezeigten Druckregelung zur Erzielung eines Soll-Tidalvolumens unmittelbar nachfolgender rein mandatorischer Atemhub weist in der Regel ein zu geringes Tidalvolumen auf bzw. ein geringeres Tidalvolumen als von der Steuerungseinrichtung 18 vorgesehen. Durch diese charakteristischen Unterschiede zur rein mandatorischen Beatmung kann die Steuerungsvorrichtung 18 ohne zusätzliche Sensoren allein aus den von den vorhandenen Flusssensoren 44 und 48 sowie den vorhandenen Drucksensoren 27 einzelne Atemhübe als aktive Atemhübe des Patienten 12 erkennen.

Wie in Figur 3 zu erkennen ist, kann jedoch alternativ oder zusätzlich ein aktiver Atemhub durch die Erfassung des Muskeldrucks im Thoraxbereich bzw. des Ösophagus-Drucks erkannt werden. Während bei rein mandatorischen Atemhüben keine oder keine nennenswerten Muskel- bzw. Ösophagus-Drücke auftreten, zeigt die Kurve 68 in Figur 3 in den aktiven Atemhüben 2 und 3 einen nennenswerten Muskel- bzw. Ösophagus-Druck, zu dessen Erfassung jedoch ein weiterer Sensor notwendig ist, der für die Beatmungsvorrichtung 10 ansonsten nicht gebraucht wird.

Die Erkennung von aktiven Atemhüben spielt für die Vermeidung falsch-positiver Fehlermeldungen an der Beatmungsvorrichtung 10 eine Rolle, wie nachfolgend erläutert werden wird.

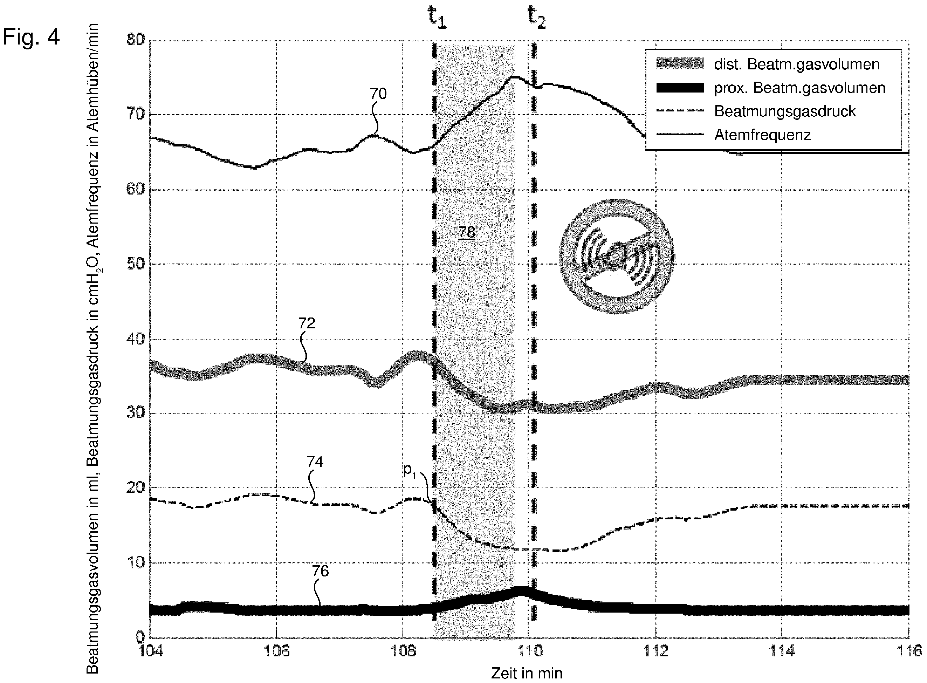

In Figur 4 ist ein erstes Diagramm gezeigt, in welchem die Atemfrequenz als Atemhübe pro Minute mit dem Graphen 70 aufgetragen ist, das distale Beatmungsgasvolumen, also das zeitliche Integral des vom distalen Durchflusssensor 48 gemessenen Beatmungsgasflusses mit der Kurve 72 in Millilitern aufgetragen ist, der Beatmungsgasdruck in Zentimeter Wassersäule als Kurve 74 und schließlich das proximale Beatmungsgasvolumen, also das zeitliche Integral des durch den proximalen Durchflusssensor 44 gemessenen Beatmungsgasflusses, als Kurve 76 wiederum in Milliliter aufgetragen ist.

Wie in der Beschreibungseinleitung dargelegt wurde, besteht üblicherweise ein betragsmäßiger Unterschied zwischen dem distalen Beatmungsgasvolumen gemäß Kurve 72 und dem proximalen Beatmungsgasvolumen gemäß Kurve 76, aufgrund von Elastizitäten der Beatmungsleitungsanordnung 30 und aufgrund von Leckageströmen (Querströmungen) im Bereich der Ventilanordnung, sodass ein Teil des Beatmungsgasstroms in der Beatmungsleitungsanordnung über das Y-Verbindungsstück 45 unmittelbar vom Inspirationsventil 20 zum Exspirationsventil 22 strömen kann, ohne den Patienten 12 zu erreichen.

In der Regel ist der betragsmäßige Unterschied zwischen distalem und proximalem Beatmungsgasvolumen bekannt bzw. ist für diesen betragsmäßigen Unterschied ein Volumen-Unterschiedswerte-Zulässigkeitsbereich definiert. Solange der betragsmäßige Unterschied zwischen distalem und proximalem Beatmungsgasvolumen in diesem Zulässigkeitsbereich liegt, besteht kein Anlass, von einer Fehlfunktion der Beatmungsvorrichtung, insbesondere vom proximalen Durchflusssensor 44, auszugehen.

Dann jedoch, wenn der betragsmäßige Unterschied zwischen dem distalen und dem proximalen Beatmungsgasvolumen den zugeordneten Zulässigkeitsbereich verlässt, könnte dies an einer funktionellen Beeinträchtigung des proximalen Durchflusssensors 44 liegen, sodass dann, wenn der genannte Volumen-Unterschiedswert den zugeordneten Zulässigkeitsbereich verlässt, die Steuereinrichtung 18 einen Fehlerkandidat-Flag setzt und somit einen Fehlerkandidaten erkennt. Dies geschieht in Figur 4 beispielsweise zum Zeitpunkt t₁.

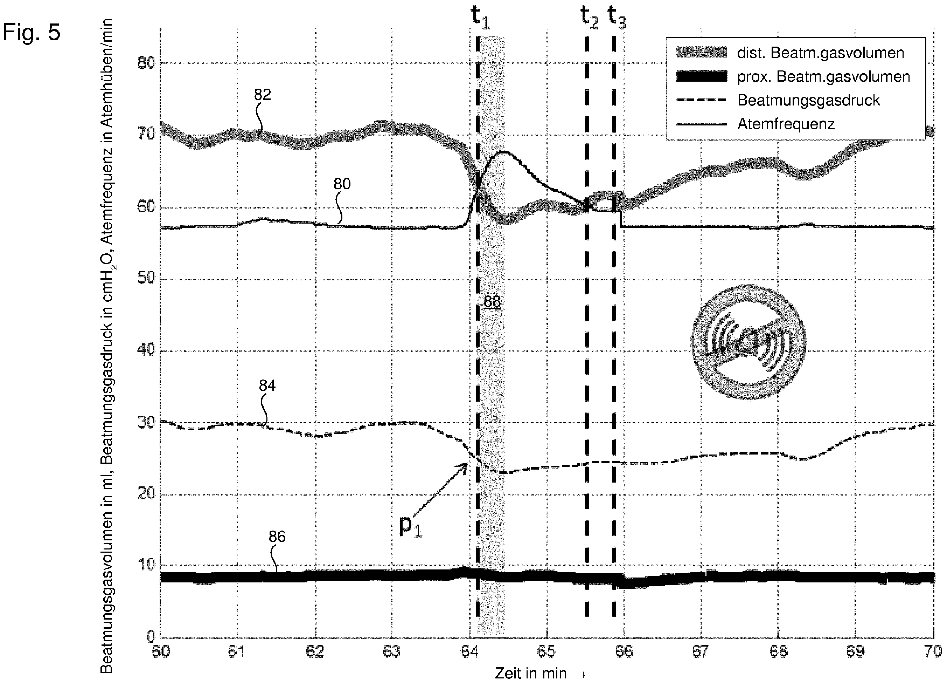

Es sei an dieser Stelle klargestellt, dass der Zeitpunkt t₁ nicht der einzige Zeitpunkt in Figur 4 sein muss, an dem die Steuereinrichtung 18 einen Kandidaten für einen Fehler des proximalen Durchflusssensors erkennt. Jedoch soll der zum Zeitpunkt t₁ erkannte Fehlerkandidat nun im Weiteren näher betrachtet werden. Mit dem Erkennen des Fehlerkandidaten zum Zeitpunkt t₁ beginnt die Steuereinrichtung eine Verifizierungsphase, die bevorzugt über genau 50 Atemhübe fortdauert. Sie endet am Zeitpunkt t₂ in Figur 4. Darüber hinaus speichert sie den zum Zeitpunkt t₁ der Erkennung des Fehlerkandidaten herrschenden Beatmungsgasdruck ab, in Figur 4 als Wert "p₁" bezeichnet. Dieser Wert p₁ ist Grundlage eines Verwerfen-Kriteriums, auf welches weiter unten im Zusammenhang mit Figur 5 näher eingegangen werden wird.

Mit Beginn der Verifizierungsphase zum Zeitpunkt t₁ beginnt die Steuereinrichtung 18, auftretende Atemhübe dahingehend zu untersuchen, ob sie aktive oder mandatorische Atemhübe sind. Sie verwendet hierzu die im Zusammenhang mit Figur 3 dargelegte Signalauswertung. Für den Beatmungsvorgang von Figur 4 ermittelt die Steuereinrichtung 18, dass der grau hinterlegte Bereich 78 durchgehend aktive Atemhübe zeigt. Da am Ende der Verifizierungsphase zum Zeitpunkt t₂ mehr als 50 % der definitionsgemäß 50 Atemhübe der Verifizierungsphase aktive Atemhübe waren, ist ein Verwerfen-Kriterium erfüllt und die Steuereinrichtung 18 verwirft den zum Zeitpunkt t₁ erkannten Fehlerkandidaten und führt auf Grundlage der Erkennung des Fehlerkandidaten keine weiteren Steuerungseingriffe durch. Hintergrund dieser Ausbildung der Steuereinrichtung 18 ist, dass in dem Fall, dass mehr als die Hälfte der Atemhübe während der Verifizierungsphase aktive Atemhübe waren, die zunächst als potenziell kritisch beurteilte Verringerung des Unterschiedswerts zwischen distalem und proximalem Beatmungsgasvolumen ihre Ursache in der Spontanatmung des Patienten 12 hat und nicht in einem Fehler oder Schaden des proximalen Durchflusssensors 44.

Im Beispiel von Figur 4 ist beispielsweise durch den Anstieg der Atemfrequenz, beginnend zum Zeitpunkt t₁, über den gesamten Spontanatmungsbereich 78 hinweg angezeigt, dass im Bereich 78 Spontanatmung des Patienten vorherrscht.

In Figur 5 ist ein Diagramm eines anderen, jedoch vergleichbaren Beatmungsvorgangs dargestellt. Gleiche Wertekurven wie in Figur 4 sind in Figur 5 mit gleichen Bezugszeichen versehen, jedoch erhöht um die Zahl 10. Wiederum liegt zum Zeitpunkt t₁ ein betragsmäßiger Unterschiedswert zwischen dem distalen und dem proximalen Beatmungsgasvolumen (siehe Kurven 82 und 86 in Figur 5) außerhalb eines Zulässigkeitsbereichs, sodass die Steuereinrichtung 18, die das Verlassen des Zulässigkeitsbereichs erkennt, eine Verifizierungsphase beginnt. Der zu Beginn der Verifizierungsphase, also zum Zeitpunkt t₁ des Erkennens eines Fehlerkandidaten herrschende Beatmungsgasdruck, gemessen durch einen mit dem proximalen Durchflusssensor 44 verbundenen Drucksensor, in einem Datenspeicher hinterlegt.

Bis zum Ende der wiederum 50 Atemhübe dauernden Verifizierungsphase zum Zeitpunkt t₂ traten zwar zu Beginn der Verifizierungsphase aktive Atemhübe auf (siehe grauer Bereich 88 in Figur 5), jedoch deutlich weniger als die Aktiv-Atemhub-Schwelle von 50 % der Atemhübe, sodass ein auf das Auftreten von aktiven Atemhüben bezogenes Verwerfen-Kriterium nicht erfüllt ist. Es ist auch kein anderes Verwerfen-Kriterium erfüllt, etwa dass der Unterschiedswert der beiden Beatmungsgasvolumina oder ein betragsmäßiger Unterschiedswert zwischen dem zum Zeitpunkt t₁ des Erkennens des Fehlerkandidaten und einem nachfolgend auftretenden Beatmungsgasdruck innerhalb eines Druck-Unterschiedswerte-Zulässigkeitsbereichs liegt.

Da jedoch in der Verifizierungsphase überhaupt Spontanatmung aufgetreten ist, verlängert die Steuereinrichtung 18 die Verifizierungsphase 12 über den Zeitpunkt t₂ hinaus, um eine vorbestimmte Zusatz-Verifizierungsphase, welche bis zum Zeitpunkt t₃ dauert und welche etwa 10 bis 15 weitere Atemhübe umfasst.

Dies bedeutet, eine Entscheidung, ob der proximale Durchflusssensor 44 als fehlerhaft eingestuft wird, wird erst nach der verlängerten Verifizierungsphase getroffen.

Bis zum Ende der Verifizierungsphase t₃ treten zwar keine weiteren aktiven Atemhübe auf, jedoch nimmt der distale Beatmungsgasfluss zu, sodass sich der betragsmäßige Unterschiedswert zwischen distalem und proximalem Beatmungsgasfluss zurück in seinen Zulässigkeitsbereich vergrößert. Somit liegt zum Zeitpunkt t₃ ein Verwerfen-Kriterium vor und der zum Zeitpunkt t₁ erkannte Fehlerkandidat wird von der Steuereinrichtung 18 verworfen. Es erfolgt auf Grundlage des zum Zeitpunkt t₁ erkannten Fehlerkandidaten kein weiterer Steuerungseingriff durch die Steuereinrichtung 18.

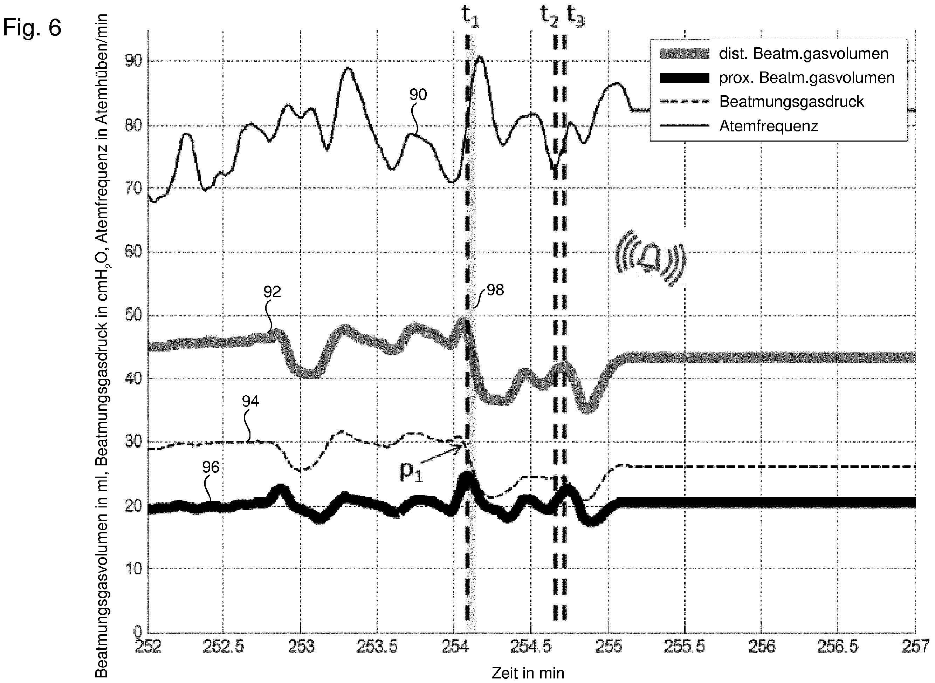

Figur 6 zeigt ein Diagramm einer weiteren, zu jenen der Figuren 4 und 5 ähnlichen Beatmungssituation. Gleiche Wertekurven wie in Figur 5 sind mit gleichen Bezugszeichen bezeichnet, jedoch erhöht um die Zahl 10.

Wiederum wird - unter anderem - zum Zeitpunkt t₁ ein Kandidat für einen Fehler des proximalen Durchflusssensors 44 durch die Steuereinrichtung 18 erkannt, da sich das distale und der proximale Beatmungsgasvolumen betragsmäßig so stark annähern, dass ihr Unterschiedswert außerhalb eines vorbestimmten Zulässigkeitsbereichs liegt. Die Steuereinrichtung 18 beginnt zum Zeitpunkt t₁ die vorbestimmte Verifizierungsphase, in welcher die Steuereinrichtung 18 prüft, ob vorbestimmte Verwerfen-Kriterien vorliegen oder eintreten, die ein Verwerfen des Fehlerkandidaten zur Folge haben.

Zu Beginn der Verifizierungsphase treten in geringem Umfang aktive Atemhübe auf, wie der schmale graue Bereich 98 anzeigt, welcher unmittelbar auf den Zeitpunkt t₁ folgt, zu welchem der Fehlerkandidat erkannt wird. Bis zum Zeitpunkt t₂, dem vorgesehenen Ende der Verifizierungsphase, ist kein Verwerfen-Kriterium eingetreten. Da jedoch in der Verifizierungsphase überhaupt Spontanatmung auftrat, wird diese um eine Zusatz-Verifizierungsphase bis zum Zeitpunkt t₃ verlängert. In dem Ausführungsbeispiel von Figur 6 ist die Zusatz-Verifizierungsphase kürzer als in dem in Figur 5 dargestellten Fall, was beispielsweise an abweichenden Patientendaten liegen kann.

Da auch bis zum Ende der verlängerten Verifizierungsphase zum Zeitpunkt t₃ kein Verwerfen-Kriterium eingetreten ist, schließt die Steuereinrichtung 18 aus dem zum Zeitpunkt t₁ erkannten Fehlerkandidaten zum Zeitpunkt t₃ auf einen Fehler und führt einen Steuerungseingriff durch, beispielsweise indem die Steuereinrichtung einen Alarm, verbunden mit einer konkreten Fehlermeldung, auslöst.

## Patentansprüche

1. Beatmungsvorrichtung (10) zur künstlichen Beatmung eines Patienten mit
- einer Beatmungsgasquelle (15),
- einer zwischen der Beatmungsgasquelle (15) und einem patientenseitigen, proximalen Ende verlaufenden Beatmungsleitungsanordnung (30),
- einer Ventilanordnung (20, 22), umfassend ein Inspirationsventil (20) und ein Exspirationsventil (22),
- einer Durchflusssensoranordnung (44, 48) zur quantitativen Erfassung eines Gasflusses in der Beatmungsleitungsanordnung (30), umfassend einen weiter vom patientenseitigen Ende der Beatmungsleitungsanordnung (30) entfernt angeordneten distalen Durchflusssensor (48) und einen näher beim patientenseitigen Ende der Beatmungsleitungsanordnung (30) gelegenen proximalen Durchflusssensor (44),
- eine Drucksensoranordnung (27) zur quantitativen Erfassung eines Gasdrucks von in der Beatmungsleitungsanordnung (30) strömendem Gas,
- einer Druckveränderungsanordnung (16) zur Veränderung des Gasdrucks des in der Beatmungsleitungsanordnung (30) strömenden Gases, und mit
- einer Steuereinrichtung (18), welche wenigstens dazu ausgebildet ist,
• den Betrieb der Druckveränderungsanordnung auf Grundlage von Messsignalen des proximalen Durchflusssensors (44) zu steuern, und
• in Abhängigkeit von Messsignalen des proximalen Durchflusssensors (44) und des distalen Durchflusssensors (48) auf einen Fehler des proximalen Durchflusssensors zu schließen,
**dadurch gekennzeichnet, dass** die Steuereinrichtung (18) dazu ausgebildet ist, in Abhängigkeit von Messsignalen des proximalen Durchflusssensors (44) und des distalen Durchflusssensors (48), und zwar aufgrund einer betragsmäßigen Annäherung der erfassten Flusswerte oder/und der daraus ermittelten Volumenwerte des proximalen Durchflusssensors (44) und des distalen Durchflusssensors (48), auf einen Fehlerkandidaten des proximalen Durchflusssensors (44) zu schließen und erst mit zeitlicher Verzögerung nach dem Erkennen des Fehlerkandidaten auf einen Fehler des proximalen Durchflusssensors (44) zu schließen, wobei die Steuereinrichtung (18) dazu ausgebildet ist, den Fehlerkandidaten zu verwerfen, wenn während einer Verifizierungsphase, welche mit oder nach dem Erkennen des Fehlerkandidaten beginnt, wenigstens ein ein Ausmaß einer Spontanatmungsaktivität des Patienten berücksichtigendes Verwerfen-Kriterium zum Verwerfen des Fehlerkandidaten erfüllt wird.

2. Beatmungsvorrichtung (10) nach Anspruch 1,
**dadurch gekennzeichnet, dass** das wenigstens eine Verwerfen-Kriterium umfasst:
- eine Anzahl von durch den mit der Beatmungsvorrichtung beatmeten Patienten ausgelösten aktiven Atemhüben des Patienten während der Verifizierungsphase übersteigt einen Aktiv-Atemhub-Schwellenwert.

3. Beatmungsvorrichtung (10) nach Anspruch 2,
**dadurch gekennzeichnet, dass** die Steuereinrichtung (18) dazu ausgebildet ist, einen aktiven Atemhub zu erkennen.

4. Beatmungsvorrichtung (10) nach Anspruch 3,
**dadurch gekennzeichnet, dass** die Steuereinrichtung (18) dazu ausgebildet ist, einen eine Zeitdauer zwischen dem Auslösen eines Erfassungsatemhubs und dem Auslösen des dem Erfassungsatemhub unmittelbar nachfolgenden Atemhubs als Atemhubdauer des Erfassungsatemhubs repräsentierenden Zeitdauer-Erfassungswert und den Zeitdauer-Erfassungswert mit einem Betriebs-Zeitdauerwert zu vergleichen, welcher eine Zeitdauer zwischen dem jeweiligen mandatorischen Auslösen von zwei unmittelbar aufeinander folgenden durch die Beatmungsvorrichtung ausgelösten mandatorischen Atemhüben als mandatorische Atemhubdauer repräsentiert, wobei die Steuereinrichtung (18) dazu ausgebildet ist, den Erfassungsatemhub auf Grundlage des Zeitdauer-Vergleichsergebnisses als aktiven Atemhub zu erkennen.

5. Beatmungsvorrichtung (10) nach Anspruch 3,
**dadurch gekennzeichnet, dass** die Steuereinrichtung (18) dazu ausgebildet ist, einen eine Zeitdauer zwischen dem Auslösen eines Erfassungsatemhubs und dem Auslösen des dem Erfassungsatemhub unmittelbar nachfolgenden Atemhubs als Atemhubdauer des Erfassungsatemhubs repräsentierenden Zeitdauer-Erfassungswert und den Erfassungswert mit einem Zeitdauer-Vergleichswert zu vergleichen, welcher eine über eine Mehrzahl von Atemhüben gemittelten Mittelwert von Atemhubdauern repräsentiert, wobei die Steuereinrichtung (18) dazu ausgebildet ist, den Erfassungsatemhub auf Grundlage des Zeitdauer-Vergleichsergebnisses als aktiven Atemhub zu erkennen.

6. Beatmungsvorrichtung (10) nach Anspruch 5,
**dadurch gekennzeichnet, dass** der Zeitdauer-Vergleichswert einen gleitenden Mittelwert von Atemhubdauern über eine vorbestimmte Anzahl von bis zum Erfassungsatemhub aufeinanderfolgenden Atemhüben wiedergibt.

7. Beatmungsvorrichtung (10) nach einem der Ansprüche 3 bis 6,
**dadurch gekennzeichnet, dass** die Steuereinrichtung (18) dazu ausgebildet ist, einen Gasdruck von in der Beatmungsleitungsanordnung (30) strömendem Gas zu erfassen und einen während eines Erfassungsatemhubs erfassten Gasdruck mit dem während eines vorhergehenden Atemhubs erfassten Gasdruck zu vergleichen, wobei die Steuereinrichtung (18) dazu ausgebildet ist, den Erfassungsatemhub auf Grundlage des Druck-Vergleichsergebnisses als aktiven Atemhub zu erkennen.

8. Beatmungsvorrichtung (10) nach einem der Ansprüche 3 bis 7,
**dadurch gekennzeichnet, dass** die Steuereinrichtung (18) dazu ausgebildet ist, während eines Erfassungsatemhubs wenigstens einen Wert eines Ösophagus-Drucks oder/und eines Pleuraldrucks des Patienten zu erfassen und auf Grundlage des wenigstens einen erfassten Druckwerts den Erfassungsatemhub als aktiven Atemhub zu erkennen.

9. Beatmungsvorrichtung (10) nach einem der Ansprüche 2 bis 8,
**dadurch gekennzeichnet, dass** der Aktiv-Atemhub-Schwellenwert wenigstens 39 %, vorzugsweise wenigstens 45 %, besonders bevorzugt wenigstens 50 % der Gesamtanzahl an Atemhüben in der Verifizierungsphase beträgt.

10. Beatmungsvorrichtung (10) nach einem der Ansprüche 2 bis 9,
**dadurch gekennzeichnet, dass** die Steuereinrichtung (18) dazu ausgebildet ist, dann wenn die während der vorbestimmten Verifizierungsphase ermittelte Anzahl an aktiven Atemhüben größer als Null und kleiner als der vorbestimmte Aktiv-Atemhub-Schwellenwert ist, die Verifizierungsphase um eine Zusatz-Verifizierungsphase zu verlängern.

11. Beatmungsvorrichtung (10) nach einem der vorhergehenden Ansprüche, **dadurch gekennzeichnet, dass** das wenigstens eine Verwerfen-Kriterium umfasst:
- ein betragsmäßiger Volumen-Unterschiedswert, welcher einen Unterschied zwischen dem mittels des distalen Durchflusssensors (48) ermittelten distalen Gasvolumen und dem mittels des proximalen Durchflusssensors (44) während desselben Atemhubs ermittelten proximalen Gasvolumens angibt, liegt innerhalb eines Volumen-Unterschiedswerte-Zulässigkeitsbereichs, oder/und
- ein betragsmäßiger Druck-Unterschiedswert, welcher einen Unterschied zwischen den während unterschiedlicher Atemhübe von der Drucksensoranordnung erfassten Gasdrücken angibt, liegt innerhalb eines Druck-Unterschiedswerte-Zulässigkeitsbereichs.

12. Beatmungsvorrichtung (10) nach einem der vorhergehenden Ansprüche, **dadurch gekennzeichnet, dass** die Steuereinrichtung (18) dazu ausgebildet ist, im bestimmungsgemäßen mandatorischen Beatmungsbetrieb die Druckveränderungsanordnung (16) zur Veränderung des Gasdrucks des in der Beatmungsleitungsanordnung (30) strömenden Gases nach Maßgabe des Messsignals (52; 52') des proximalen Durchflusssensors (44) derart anzusteuern, dass ein auf Grundlage des vom proximalen Durchflusssensor (44) erfassten Gasflusswert ermittelter Gasvolumenwert innerhalb eines vorgegebenen Volumen-Wertebereichs liegt, vorzugsweise im Wesentlichen konstant ist.

13. Beatmungsvorrichtung (10) nach einem der vorhergehenden Ansprüche, **dadurch gekennzeichnet, dass** der proximale Durchflusssensor (44) ein Differenzdrucksensor (44) ist, wobei die Drucksensoranordnung (27) wenigstens einen der am Differenzdrucksensor (44) erfassten Gasdrücke als den Gasdruck von in der Beatmungsleitungsanordnung (30) strömendem Gas erfasst und dass die Steuereinrichtung (18) diesen erfassten Gasdruck zur Ermittlung eines Fehlerkandidaten des proximalen Durchflusssensors (44) verwendet.

14. Beatmungsvorrichtung (10) nach einem der vorhergehenden Ansprüche, **dadurch gekennzeichnet, dass** die Steuereinrichtung (18) dazu ausgebildet ist, dann, wenn sie auf einen Fehler in dem proximalen Durchflusssensor (44) schließt, einen Steuerungseingriff an der Beatmungsvorrichtung (10) vorzunehmen, wie etwa eine Fehlermeldung oder einen Alarm auszugeben.

## Claims

1. A ventilation apparatus (10) for artificial ventilation of a patient, having
- a respiratory gas source (15);
- a ventilation conduit arrangement (30) proceeding between the respiratory gas source (15) and a patient-side proximal end;
- a valve arrangement (20, 22) encompassing an inhalation valve (20) and an exhalation valve (22);
- a flow sensor arrangement (44, 48) for quantitative detection of a gas flow in the ventilation conduit arrangement (30), encompassing a distal flow sensor (48) arranged farther from the patient-side end of the ventilation conduit arrangement (30), and a proximal flow sensor (44) located closer to the patient-side end of the ventilation conduit arrangement (30);
- a pressure sensor arrangement (27) for quantitative detection of a gas pressure of gas flowing in the ventilation conduit arrangement (30);
- a pressure modification arrangement (16) for modifying the gas pressure of the gas flowing in the ventilation conduit arrangement (30); and having
- a control device (18) that is embodied at least
• to control the operation of the pressure modification arrangement on the basis of measured signals of the proximal flow sensor (44), and
• to infer a fault in the proximal flow sensor as a function of measured signals of the proximal flow sensor (44) and of the distal flow sensor (48),
**characterized in that** the control device (18) is embodied to infer, as a function of measured signals of the proximal flow sensor (44) and of the distal flow sensor (48), namely based on the detected flow values and/or on the volume values determined therefrom of the proximal flow sensor (44) and the distal flow sensor (48) approaching each other according to amount, a candidate fault in the proximal flow sensor (44), and to infer a fault in the proximal flow sensor (44) only with a time delay after identification of the candidate fault, the control device (18) being embodied to reject the candidate fault if, during a verification phase that begins with or after identification of the candidate fault, at least one rejection criterion, taking into account a degree of spontaneous respiration activity by the patient, for rejecting the candidate fault is met.

2. The ventilation apparatus (10) according to Claim 1,
**characterized in that** the at least one rejection criterion encompasses:
- a number of active breaths by the patient being ventilated with the ventilation apparatus which are initiated by the patient during the verification phase exceeds an active-breath threshold value.

3. The ventilation apparatus (10) according to Claim 2,
**characterized in that** the control device (18) is embodied to identify an active breath.

4. The ventilation apparatus (10) according to Claim 3,
**characterized in that** the control device (18) is embodied to detect a time period detection value, which represents a time period between the initiation of a detected breath and the initiation of the breath immediately following initiation of the detected breath, as a breath duration of the detected breath, and to compare the time period detection value with an operating time period value that represents a time period between the respective mandatory initiations of two immediately successive mandatory breaths initiated by the ventilation apparatus, constituting a mandatory breath duration, the control device (18) being embodied to identify the detected breath, on the basis of the time period comparison result, as an active breath.

5. The ventilation apparatus (10) according to Claim 3,
**characterized in that** the control device (18) is embodied to detect a time period detection value, which represents a time period between the initiation of a detected breath and the initiation of the breath immediately following the detected breath, as a breath duration of the detected breath, and to compare the detected value with a time period comparison value that represents an average value of breath durations averaged over a plurality of breaths, the control device (18) being embodied to identify the detected breath as an active breath on the basis of the time period comparison result.

6. The ventilation apparatus (10) according to Claim 5,
**characterized in that** the time period comparison value reproduces a sliding average of breath durations over a predetermined number of breaths following one another until the detected breath.

7. The ventilation apparatus (10) according to one of Claims 3 to 6, **characterized in that** the control device (18) is embodied to detect a gas pressure of gas flowing in the ventilation conduit arrangement (30), and to compare a gas pressure detected during a detected breath with the gas pressure detected during a preceding breath, the control device (18) being embodied to identify the detected breath as an active breath on the basis of the pressure comparison result.

8. The ventilation apparatus (10) according to one of Claims 3 to 7, **characterized in that** the control device (18) is embodied to detect, during a detected breath, at least one value of an esophageal pressure and/or of a pleural pressure of the patient, and to identify the detected breath as an active breath on the basis of the at least one detected pressure value.

9. The ventilation apparatus (10) according to one of Claims 2 to 8, **characterized in that** the active breath threshold value is equal to at least 39%, preferably at least 45%, particularly preferably at least 50%, of the total number of breaths in the verification phase.

10. The ventilation apparatus (10) according to one of Claims 2 to 9, **characterized in that** the control device (18) is embodied to extend the verification phase to include an additional verification phase when the number of active breaths ascertained during the predetermined verification phase is greater than zero and less than the predetermined active breath threshold value.

11. The ventilation apparatus (10) according to one of the preceding claims, **characterized in that** the at least one rejection criterion additionally encompasses:
- a quantitative volume difference value, which indicates a difference between the distal gas volume ascertained by means of the distal flow sensor (48) and the proximal gas volume ascertained by means of the proximal flow sensor (44) during the same breath, lies within a volume difference value permissibility range; and/or
- a quantitative pressure difference value, which indicates a difference between the gas pressures detected by the pressure sensor arrangement during different breaths, lies within a pressure difference value permissibility range.

12. The ventilation apparatus (10) according to one of the preceding claims, **characterized in that** herein the control device (18) is embodied to control the pressure modification arrangement (16), in the mandatory ventilation operating mode as intended, to modify the gas pressure of the gas flowing in the ventilation conduit arrangement (30) on the basis of the measured signal (52; 52') of the proximal flow sensor (44) in such a way that a gas volume value ascertained on the basis of the gas flow value detected by the proximal flow sensor (44) lies within a predefined volume value range, preferably is substantially constant.

13. The ventilation apparatus (10) according to one of the preceding claims, **characterized in that** herein the proximal flow sensor (44) is a differential pressure sensor (44), the pressure sensor arrangement (27) detecting at least one of the gas pressures detected at the differential pressure sensor as the gas pressure of gas flowing in the ventilation conduit arrangement (30); and the control device (18) uses that detected gas pressure to ascertain a candidate fault in the proximal flow sensor (44).

14. The ventilation apparatus (10) according to one of the preceding claims, **characterized in that** herein the control device (18) is embodied to perform a control action on the ventilation apparatus (10), for example to output a fault message or an alarm, when it infers a fault in the proximal flow sensor (44).

## Revendications

1. Dispositif de ventilation (10) pour la ventilation artificielle d'un patient avec
- une source de gaz de ventilation (15),
- un agencement de conduites de ventilation (30) s'étendant entre la source de gaz de ventilation (15) et une extrémité proximale côté patient,
- un ensemble de soupapes (20, 22) comprenant une soupape d'inspiration (20) et une soupape d'expiration (22),
- un agencement de capteurs de débit (44, 48) pour la détection quantitative d'un flux de gaz dans l'agencement de conduites de ventilation (30), comprenant un capteur de débit distal (48) disposé plus loin de l'extrémité côté patient de l'agencement de conduites de ventilation (30) et un capteur de débit proximal (44) situé plus près de l'extrémité côté patient de l'agencement de conduites de ventilation (30),
- un agencement de capteurs de pression (27) pour la détection quantitative d'une pression de gaz s'écoulant dans l'agencement de conduites de ventilation (30),
- un agencement de modification de pression (16) pour modifier la pression de gaz du gaz s'écoulant dans l'agencement de conduites de ventilation (30), et avec
- un dispositif de commande (18) qui est adapté au moins pour
• commander le fonctionnement de l'agencement de modification de la pression sur la base de signaux de mesure du capteur de débit proximal (44), et
• conclure à une erreur du capteur de débit proximal en fonction des signaux de mesure du capteur de débit proximal (44) et du capteur de débit distal (48),
**caractérisé en ce que** le dispositif de commande (18) est adapté pour, en fonction de signaux de mesure du capteur de débit proximal (44) et du capteur de débit distal (48), et ce sur la base d'un rapprochement en valeur absolue des valeurs de débit saisies ou/et des valeurs de volume du capteur de débit proximal (44) et du capteur de débit distal (48) qui en sont déterminées, conclure à un candidat à l'erreur du capteur de débit proximal (44) et de conclure à une erreur du capteur de débit proximal (44) seulement avec un retard temporel après la reconnaissance du candidat à l'erreur, le dispositif de commande (18) étant adapté à cet effet, pour rejeter le candidat à l'erreur si, pendant une phase de vérification qui commence avec ou après la détection du candidat à l'erreur, au moins un critère de rejet prenant en compte un degré d'activité respiratoire spontanée du patient est rempli pour rejeter le candidat à l'erreur.

2. Dispositif de ventilation (10) selon la revendication 1,
**caractérisé en ce que** ledit au moins un critère de rejet comprend :
- un nombre de courses respiratoires actives du patient déclenchées par le patient ventilé par le dispositif de ventilation pendant la phase de vérification est supérieur à un seuil de courses respiratoires actives.

3. Dispositif de ventilation (10) selon la revendication 2,
**caractérisé en ce que** le dispositif de commande (18) est adapté pour détecter une course respiratoire active.

4. Dispositif de ventilation (10) selon la revendication 3,
**caractérisé en ce que** le dispositif de commande (18) est adapté pour comparer une valeur de détection de durée représentant une durée entre le déclenchement d'une course respiratoire de détection et le déclenchement de la course respiratoire suivant immédiatement la course respiratoire de détection comme durée de course respiratoire de la course respiratoire de détection et la valeur de détection de durée avec une valeur de durée de fonctionnement, laquelle représente une durée entre le déclenchement mandatoire respectif de deux courses respiratoires mandatoires déclenchées immédiatement l'une après l'autre par le dispositif de ventilation, en tant que durée de course respiratoire mandatoire, le dispositif de commande (18) étant adapté pour reconnaître la course respiratoire de détection en tant que course respiratoire active sur la base du résultat de la comparaison des durées.

5. Dispositif de ventilation (10) selon la revendication 3,
**caractérisé en ce que** le dispositif de commande (18) est adapté pour comparer une valeur de détection de durée représentant une durée entre le déclenchement d'une course respiratoire de détection et le déclenchement de la course respiratoire suivant immédiatement la course respiratoire de détection en tant que durée de course respiratoire de la course respiratoire de détection, et pour comparer la valeur de détection à une valeur de comparaison de durée, laquelle représente une valeur moyenne de durées de course respiratoire calculée sur une pluralité de courses respiratoires, le dispositif de commande (18) étant adapté pour reconnaître la course respiratoire de détection comme course respiratoire active sur la base du résultat de la comparaison des durées.

6. Dispositif de ventilation (10) selon la revendication 5,
**caractérisé en ce que** la valeur de comparaison de durée représente une moyenne mobile de durées de course respiratoire sur un nombre prédéterminé de courses respiratoires successives jusqu'à la course respiratoire de détection.

7. Dispositif de ventilation (10) selon l'une des revendications 3 à 6, **caractérisé en ce que** le dispositif de commande (18) est adapté pour détecter une pression de gaz du gaz circulant dans l'ensemble de conduites de ventilation (30) et pour comparer une pression de gaz détectée pendant une course respiratoire de détection à la pression de gaz détectée pendant une course respiratoire précédente, le dispositif de commande (18) étant adapté pour reconnaître la course respiratoire de détection comme une course respiratoire active sur la base du résultat de la comparaison de pression.

8. Dispositif de ventilation (10) selon l'une des revendications 3 à 7, **caractérisé en ce que** le dispositif de commande (18) est adapté pour détecter, pendant une course respiratoire de détection, au moins une valeur d'une pression œsophagienne ou/et d'une pression pleurale du patient et pour reconnaître, sur la base de ladite au moins une valeur de pression détectée, la course respiratoire de détection comme une course respiratoire active.

9. Dispositif de ventilation (10) selon l'une des revendications 2 à 8, **caractérisé en ce que** le seuil de course respiratoire active représente au moins 39 %, de préférence au moins 45 %, de manière particulièrement préférée au moins 50 % du nombre total de courses respiratoires dans la phase de vérification.

10. Dispositif de ventilation (10) selon l'une des revendications 2 à 9, **caractérisé en ce que** le dispositif de commande (18) est adapté pour, lorsque le nombre de courses respiratoires actives déterminé pendant la phase de vérification prédéterminée est supérieur à zéro et inférieur au seuil de course respiratoire active prédéterminée, prolonger la phase de vérification d'une phase de vérification supplémentaire.

11. Dispositif de ventilation (10) selon l'une des revendications précédentes, **caractérisé en ce que** ledit au moins un critère de rejet comprend :
- une valeur de différence de volume en valeur absolue, qui indique une différence entre le volume de gaz distal déterminé au moyen du capteur de débit distal (48) et le volume de gaz proximal déterminé au moyen du capteur de débit proximal (44) pendant la même course respiratoire, se situe à l'intérieur d'une plage d'admissibilité de valeurs de différence de volume, ou/et
- une valeur de différence de pression en valeur absolue, qui indique une différence entre les pressions de gaz détectées par l'agencement de capteurs de pression pendant différentes courses respiratoires, se situe dans une plage d'admissibilité de valeurs de différence de pression.

12. Dispositif de ventilation (10) selon l'une des revendications précédentes, **caractérisé en ce que** le dispositif de commande (18) est adapté pour, en mode de ventilation mandatoire conforme, activer l'agencement de modification de pression (16) pour modifier la pression du gaz circulant dans l'agencement des conduites de ventilation (30) en fonction du signal de mesure (52 ; 52') du capteur de débit proximal (44) de telle sorte qu'une valeur de volume de gaz déterminée sur la base de la valeur de débit de gaz détectée par le capteur de débit proximal (44) se situe à l'intérieur d'une plage de valeurs de volume prédéfinie, de préférence soit sensiblement constante.

13. Dispositif de ventilation (10) selon l'une des revendications précédentes, **caractérisé en ce que** le capteur de débit proximal (44) est un capteur de pression différentielle (44), l'ensemble de capteurs de pression (27) détectant au moins l'une des pressions de gaz détectées au niveau du capteur de pression différentielle (44) en tant que pression de gaz s'écoulant dans l'ensemble de conduites de ventilation (30), et **en ce que** le dispositif de commande (18) utilise cette pression de gaz détectée pour déterminer une erreur candidate du capteur de débit proximal (44).

14. Dispositif de ventilation (10) selon l'une quelconque des revendications précédentes,
**caractérisé en ce que** le dispositif de commande (18) est adapté pour, lorsqu'il conclut à un défaut du capteur de débit proximal (44), effectuer une intervention de commande sur le dispositif de ventilation (10), telle qu'un message de défaut ou une alarme.
